# EUROPEAN PATENT SPECIFICATION

(11) **EP 0 609 905 B1**
(45) Date of publication and mention of the grant of the patent: **06.06.2001**
(21) Application number: 94102762.5
(22) Date of filing: 22.08.1989
(51) Int. Cl.: C07D 413/04, C07D 413/14

(54) **3-(Nitrogen substituted)phenyl-5beta-amidomethyloxazolidin-2-ones**
3-(Stickstoff substituierte)phenyl-5-beta-amidomethyloxazoliden-2-one
3-Phényl azoté-5-bêta-amidométhyl-oxazolidine-2-ones

(30) Priority: 15.09.1988 US 244988; 05.10.1988 US 253850; 17.03.1989 US 324942
(43) Date of publication of application: 10.08.1994
(62) Divisional of application: 89308506.8
(73) Proprietor: PHARMACIA & UPJOHN COMPANY, Kalamazoo, Michigan 49001 (US)
(72) Inventor: Brickner, Steven L., Portage, Michigan 49081 (US)
(74) Representative: Perry, Robert Edward

(56) References cited:
- EP-A- 0 127 902
- EP-A- 0 184 170
- EP-A- 0 311 090

## Description

The present invention relates to 3-(nitrogen-substituted)phenyl-5β-amidomethyloxazolidinones which are useful as antibacterial agents.

### Background of the Invention

US-A-4128654 discloses 5-halomethylphenyl-2-oxazolidinones which are useful in controlling fungal and bacterial diseases of plants.

US-A-4250318 discloses 3-substituted phenyl-5-hydroxymethyloxazolidinones having antidepressant utility.

US-B-0029607 discloses 3-substituted phenyl-5-hydroxymethyloxazolidinones having antidepressant, tranquillising and sedative utility.

US-A-4340606 discloses 3-(p-alkylsulfonyl)phenyl-5-(hydroxymethyl or acyloxymethyl)oxazolidinones having antibacterial activity in mammals.

BE-A-0892270 discloses 3-(arylalkyl, arylalkenyl or arylacetylenic-substituted phenyl)-5-(aminomethyl)-oxazolidinones which are inhibitors of monoamine oxidase.

US-A-4461773 discloses 3-substituted phenyl-5-hydroxymethyloxazolidinones which have antibacterial activity.

EP-A-0127902 and EP-A-0184170 disclose 3-substituted phenyl-5-amidomethyloxazolidinones which have antibacterial utility.

EP-A-0311090 (= US-A-4705799) discloses aminomethyloxo-oxazolidinyl benzene derivatives including sulfides, sulfoxides, sulfones and sulfonamides which possess antibacterial activity.

US-A-4801600 discloses 6'-indolinyloxazolidinones.

### Summary of the Invention

Disclosed are 3-(nitrogen substituted)phenyl-5β-(amidomethyl)-oxazolidin-2-ones of formula (LV) where
(I) R₁ is -H,
   C₁-C₁₂ alkyl optionally substituted with 1-3 Cl,
   C₃-C₁₂ cycloalkyl,
   C₅-C₁₂ alkenyl containing one double bond,
   -φ optionally substituted with 1-3 -OH, -OCH₃, -OC₂H₅, -NO₂, -F, -Cl, -Br, -COOH and -SO₃H, -N(R₁₋₁)(R₁₋₂) where R₁₋₁ and R₁₋₂ are the same or different and are -H, C₁-C₂ alkyl,
   furanyl,
   tetrahydrofuranyl,
   2-thiophene,
   pyrrolidinyl,
   pyridinyl,
   -O-R₁₋₃ where R₁₋₃ is C₁-C₄ alkyl,
   -NH₂,
   -NHR₁₋₃ where R₁₋₃ is as defined above,
   -NR₁₋₃R₁₋₄ where R₁₋₄ is C₁-C₃ alkyl and R₁₋₃ is as defined above, and where R₁₋₃ and R₁₋₄ can be taken together with the attached nitrogen atom to form a saturated mono-nitrogen C₅-C₇ heterocyclic ring including -O- (morpholine),
   -CH₂-OH,
   -CH₂-OR₁₋₅ where R₁₋₅ is C₁-C₄ alkyl or -CO-R₁₋₆ where R₁₋₆ is C₁-C₄ alkyl or -φ;
(II) two of R₂, R₃ and R₄ are -H and the other of R₂, R₃ and R₄ is -H,
   -F, -Cl, Br, -I,
   C₁-C₆ alkyl,
   -OH,
   -CO-O-R₂₋₁ where R₂₋₁ is C₁-C₄ alkyl or -φ,
   -O-R₂₋₁ where R₂₋₁ is as defined above,
   -COOH,
   -COO⁻,
   -CHO,
   -CO-NR₂₋₂R₂₋₃ where R₂₋₂ and R₂₋₃ are the same or different and are -H, C₁-C₃ alkyl, or R₂₋₂ and R₂₋₃ are taken together with the attached nitrogen atom to form a saturated mono-nitrogen C₃-C₆ heterocyclic ring optionally containing -O-,
   -C≡N,
   -C≡CH,
   -N≡C,
   -CHOH-CH₃,
   -CO-CH₃,
   -SH,
   -SO-CH₃,
   -SO-φ,
   -S-CH₃,
   -S-φ,
   -SO₂-CH₃,
   -SO₂-φ,
   -SO₃H,
   -SO₂-NH₂,
   -N₃,
   -NO₂,
   -NR_{2-4R2-5} where R₂₋₄ and R₂₋₅ are the same or different and are -H and C₁-C₃ alkyl,
   -NH-CO-R₂₋₆ where R₂₋₆ is C₁-C₄ alkyl or -φ,
   -NH-CO-NH₂,
   -CH=CH₂,
   -CH₂-CH=CH₂,
   -CH=N-OH,
   -CH=N-OCH₃, 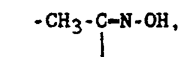 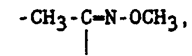
   -C*H-CH₂-O* where the atoms marked with the asterisk (^{*}) are bonded to each other resulting in the formation of a ring,
(IIIA) where W₁ and W₂ taken together are

   -NR₅-N=CR₆- (XXXII)

   where R₅ is -H,
   C₁-C₁₂ alkyl,
   -CH₂-φ,
   -CH₂CH₂-φ,
   C₃-C₇ cycloalkyl,
   C₂-C₁₂ alkenyl containing from 1 thru 3 double bonds,
   C₂-C₁₂ alkynyl containing 1 triple bond,
   -CHO,
   -CO-R₅₋₁ where R₅₋₁ is
      (A) C₁-C₆ alkyl optionally substituted with 1 -O-CH₃, -COOH, -NH₂, -SO₃H or 1-3 -Cl,
      (B) C₃-C₇ cycloalkyl,
      (C) 2-pyridinyl, 2-thiophene, 2-thiophenemethyl or 2-pyrrole,
      (D) -φ optionally substituted with 1-3
         -F, -Cl, Br, -I,
         C₁-C₆ alkyl,
         -OH,
         -CO-O-R₅₋₂ where R₅₋₂ is C₁-C₄ alkyl or -φ,
         -O-R₅₋₂ where R₅₋₂ is as defined above,
         -COOH,
         -CO-NR₅₋₃R₅₋₄ where R₅₋₃ and R₅₋₄ are the same or different and are -H, C₁-C₃ alkyl, or R₅₋₃ and R₅₋₄ are taken together with the attached nitrogen atom to form a saturated mono-nitrogen C₃-C₆ heterocyclic ring optionally containing -O-,
         -C≡N,
         -CHOH-CH₃,
         -CO-CH₃,
         -SH,
         -SO-CH₃,
         -SO-φ,
         -S-CH₃,
         -S-φ,
         -SO₂-CH₃,
         -SO₂-φ,
         -SO₃H,
         -SO₂-NH₂,
         -N₃,
         -NO₂,
         -NR_{5-5R5-6} where R₅₋₅ and R₅₋₆ are the same or different and are -H and C₁-C₃ alkyl,
         -NH-CO-R₅₋₇ where R₅₋₇ is C₁-C₄ alkyl or -φ,
   -CO-O-R₅₋₈ where R₅₋₈ is C₁-C₄ alkyl or -φ either optionally substituted with 1 or 2
      -F, -Cl, Br, -I,
      C₁-C₆ alkyl,
      -OH,
      -CO-O-R₅₋₂ where R₅₋₂ is as defined above,
      -O-R₅₋₂ where R₅₋₂ is as defined above,
      -COOH,
      -CO-NR₅₋₃R₅₋₄ where R₅₋₃ and R₅₋₄ are as defined above,
      -C≡N,
      -CHOH-CH₃,
      -CO-CH₃,
      -SH,
      -SO-CH₃,
      -SO-φ,
      -S-CH₃,
      -S-φ,
      -SO₂-CH₃,
      -SO₂-φ,
      -SO₃H,
      -SO₂-NH₂,
      -N₃,
      -NO₂,
      -NR_{5-5R5-6} where R₅₋₅ and R₅₋₆ are as defined above,
      -NH-CO-R₅₋₇ where R₅₋₇ is as defined above,
   -CO-N(R₅₋₉)₂ where R₅₋₉ is -H or R₅₋₈ as defined above and where the R₅₋₉'s can be taken together with the attached nitrogen atom to form a saturated mononitrogen C₃-C₆ heterocyclic ring optionally containing -O- or -NH-,
   -CO-CH₂-CN,
   -CO-CH₂-OH,
   -CO-CH₂-O-φ where φ is optionally substituted with 1-3 -O-CH₃, 1 -NO₂ and 1 -NH₂,
   -CO-CH₂-O-R₅₋₁₀ where R₅₋₁₀ is C₁-C₆ alkyl,
      -φ optionally substituted with 1-3 -O-CH₃, 1 -NO₂ and -NH₂,
      -CO-R₅₋₁₁ where R₅₋₁₁ is C₁-C₆ alkyl, -φ optionally substituted with 1-4 -F, 1-3 -Cl, 1 -OCH₃, -OH, -NH₂, -NO₂, -CO-CH₃, -SO₂-CH₃ and -SO₂-OH,
   -CO-CH(NH-CO-R₅₋₁₂)-R₅₋₁₃ where R₅₋₁₃ is -H or -CH₃ and R₅₋₁₂ is C₁-C₆ alkyl, -φ optionally substituted with 1 or 2 -OH, -OCH₃, -NO₂, -NH₂, -Cl, -F, -NH-CH3 and -N(CH₃)₂,
   -CO-CHNH₂-R₅₋₁₄ where R₅₋₁₄ is -CH(CH₃)₂, -CH₂-CH(CH₃)₂, -CHCH₃-CH₂-CH₃, -CH₂-OH, -CH(OH)-CH₃, -CH₂-φ, -CH₂-φ-OH, -CH₂-SH, -CH₂-CH₂-S-CH₃, and -CH₂-COOH,
   -SO₂-CH₃,
   -SO₂-φ,
   -SO₃H,
   and R₆ is -H and C₁-C₃ alkyl; or
(IIIB) where W₁ and W₂ taken together are

   -NR₅-N=N- (LIV)

   where R₅ is as defined above;
   and pharmaceutically acceptable salts thereof.

When R₅ is not H, compounds of the invention may be used for the manufacture of a medicament for use in the treatment of infection caused by gram-positive or anaerobic organisms. When R₅ is H, the compounds are useful as intermediates in the preparation of active compounds.

### DETAILED DESCRIPTION OF THE INVENTION

Compounds of the invention in which W₁ and W₂ together are -NR₅-N=CR₆- are indazolyloxazolidin-2-ones; see formula XXXII in Chart A. Compounds of the invention in which W₁ and W₂ together are -NR₅-N=N- are benzotriazolyloxazolidin-2-ones; see formula LIV in Chart B.

The synthesis of the indazolyloxazolidin-2-ones (XXXII) starts with the appropriate nitroindazole (XXII). It is preferred that R₂, R₃ and R₄ all be -H. It is preferred that R₆ is -H or -CH₃. The indazolyl nitrogen of the nitroindazoles (XXII) is protected, as previously discussed, to produce the corresponding protected nitroindazoles (XXIII). It is preferred that X₁ be t-butyloxycarbonyl. Next, the nitro group of the protected nitroindazoles (XXIII) is reduced with hydrogen, as previously discussed, to the corresponding protected aminoindazoles (XXIV). Acylation of free unprotected amino group of the protected aminoindazoles (XXIV) with a carbobenzyloxy (CBZ) group gives the urethanes (XXV). The urethanes (XXV) are then reacted with Br-CH₂-CH-CH₂ in THF and a base, as previously discussed, forming the protected allyl compounds (XXVI) and the bisallyl compounds (XXVI'). The protected allyl compounds (XXVI) and the bisallyl compounds (XXVI') can be separated at this point but it is preferrable to use the mixture as the starting material for the next step. The protected allyl compounds (XXVI) and the bisallyl compounds (XXVI') are cyclized to form the oxazolidinone nucleus by reaction with an electrophilic agent, as previously discussed. The oxazolidinone nuclei formed are the protected iodomethyloxazolidin-2-ones (XXVII) and the allyliodomethyloxazolidin-2-ones (XXVII'). Following formation of the oxazolidinone ring, the desired side chain at the 5-position is formed, as previously discussed, to form the azides (XXVIII) and allylazides (XXVIII'). During the reaction of the iodomethyl compounds (XXVII/XXVII') to the corresponding azides (XXVIII/XXVIII') the protecting group, X₁, of the iodomethyl compounds (VI) may be lost, see CHART A. In other cases it will be retained and can be removed after the aminomethyl group is acylated. The azides (XXVIII) and allylazides (XXVIII') can be separated but it is preferred to not separate them at this stage but to reduce the mixture. The azides (XXVIII) and allylazides (XXVIII') are reduced with hydrogen, as previously discussed, to give aminomethyloxazolidin-2-ones (XXIX) and 3-allyl-5-aminomethyloxazolidin-2-ones (XXIX').

When the oxazolidinone nucleus is formed to give compounds (XXVII and XXVII') an asymmetric center is created at C₅ which gives rise to a racemic mixture. It is preferrable to resolve the racemic mixture, if desired, at the aminomethyloxazolidin-2-one (XXIX) and allylaminomethyloxazolidin-2-one (XXIX') stage using methods known to those skilled in the art, as previously discussed.

The aminomethyloxazolidin-2-ones (XXIX) and allylaminomethyloxazolidin-2-ones (XXIX') are then acylated to produce the protected indazolyloxazolidin-2-ones (XXX), unprotected indazolyloxazolidin-2-ones (XXXI) and allyl indazolyloxazolidin-2-ones (XXX') containing the desired R₁ group at C₅. In the case of the indazolyloxazolidin-2-ones (XXX) the acylation produces the bis acylated compound with the acyl group also at the 1-indazolyl position. In most cases this will be a desired product and therefore is in the scope of the claimed indazolyloxazolidin-2-ones (XXXII). The allyl indazolyl-oxazolidin-2-ones (XXXII') are useful pharmacological agents and intermediates within the scope of the indazolyloxazolidin-2-ones (XXXII). It is preferred that R₁ is H, C₁-C₆ alkyl, C₃-C₆ cycloalkyl, -OCH₃ and -CHCl₂; it is more preferred that R₁ is -CH₃.

In the cases where the protecting group, X₁, was not cleaved by azide the protecting group is then removed, for example, by trifluoroacetic acid treatment to give the unprotected indazolyloxazolidin-2-ones (XXXI).

The unprotected indazolyloxazolidin-2-ones (XXXI) are then N-acylated or N-alkylated, if necessary, with the desired R₅ group, either as the acid halide, anhydride, or alkyl halide to produce the desired indazolyloxazolidin-2-ones (XXXII). For the indazolyloxazolidin-2-ones (XXXII), it is preferred that R5 is selected from the group consisting of -CH₃, -CH₂-CH=CH₂, -CH₂-C≡CH, -CHO, -CO-R₅₋₁ where R₅₋₁ is -CH₃, -C₂H₅, -CH(CH₃)₂, -CHCl₂, -CH₂-OH, -CH₂-O-CH₃, 2-thiophene and cyclopentyl. It is more preferred that R₅ is -CH₃, -CH₂-CH-CH₂, -CHO, -CO-R₅₋₁ where R₅₋₁ is -CH₃, -C₂H₅, -CHCl₂, -CH₂-CN, -CH₂-OH, -CH₂-O-CH₃, -CH₂-O-CO-CH₃, -CH₂-O-CH₂-φ or 2-thiophene.

The benzotriazolyloxazolidin-2-ones (LIV) are prepared in a similar manner (compare CHART B) to the indazolyloxazolidin-2-ones (XXXII) (CHART A) with the following exceptions. First, with the indazolyl compounds when transforming the urethane (XXV) to the protected allyl compound (XXVI), the allyl group replaced the protecting group (X₁) to some extent producing the bisallylindazolyl compounds (XXVI'); with the benzotriazolyloxazolidin-2-ones (LIV) the X₁ protecting group is not lost when transforming the urethanes (XLVII) to the protected compounds (XLVIII). Second, with the indazolyl compounds when reducing the azide (XXVIII) with hydrogen again the protecting group is lost producing the aminomethyl compounds (XXIX); with the benzotriazolyloxazolidin-2-ones (LIV) the protecting group X₁ is not lost when reducing the protected azides (L).

The indazolyloxazolidin-2-ones (XXXII) and the benzotriazolyloxazolidin-2-ones (LIV) all have an asymmetric center at the C₅-position of the oxazolidinone ring which produces two enantiomers. The mixture of enantiomers is resolved by means known to those skilled in the art. The enantiomer which is pharmacologically active is the β-enantiomer, see CHARTS A and B. The racemic mixture is useful in the same way and for the same purpose as the pure β-enantiomer; the difference is that twice as much racemic material must be used to produce the same effect as the pure β-enantiomer.

For convience the indazolyloxazolidin-2-ones (XXXII) and benzotriazolyloxazolidin-2-ones (LIV) will be collectively referred to as the 3-(nitrogen substituted)phenyl-5β-amidomethyloxazolidin-2-ones (LV).

The 3-(nitrogen substituted)phenyl-5β-(amidomethyl)oxazolidin-2-ones (LV) of the present invention are useful as antibacterial agents in treating infections in mammals caused by gram-positive and anaerobic infections. It is preferred to treat humans and useful warm-blooded mammals such as cattle, horses, sheep, hogs, dogs, cats, etc.

The 3-(nitrogen substituted)phenyl-5β-(amidomethyl)oxazolidin-2-ones (LV) of the present invention are also useful in treating AIDS patients infected with *Mycobacterium avium*.

The 3-(nitrogen substituted)phenyl-5β-(amidomethyl)oxazolidin-2-ones (LV) can be administered either parenterally (IV, IM, SQ) or orally. The daily dose is about 5 to about 20 mg/kg. This dose can preferrably be given in divided doses and administered 2-4 times daily. The preferred route of administration as well as the particular dosage form for either the parenteral or oral route depends on the particular facts of the situation including the nature of the infection and condition of the patient. The usual pharmaceutical dosage forms appropriate for parenteral (solution, suspension in oil) and oral (tablet, capsule, syrup, suspension, etc) administration are known to those skilled in the art and there is nothing unusual about using those dosage forms with the 3-(nitrogen substituted)phenyl-5β-(amidomethyl)oxazolidin-2-ones (LV). The exact dosage of the 3-(nitrogen substituted)phenyl-5β-(amidomethyl)oxazolidin-2-ones (LV) to be administered, the frequency of administration, route of administration, the dosage form will vary depending on a number of factors known to those skilled in the art including the age, weight, sex, general physical condition of the patient, the nature of the infection (particular microorganism involved, its virulence, the extent of the infection) other medical problems of the patient, etc as is well known to the physican treating infectious diseases.

The 3-(nitrogen substituted)phenyl-5β-(amidomethyl)oxazolidin-2-ones (LV) can be used either alone or in conjunction with other antibacterial agents as is known to those skilled in the art. Further, the 3-(nitrogen substituted)phenyl-5β-(amidomethyl)oxazolidin-2-ones (LV) can be used in conjunction with non-antibacterial agents as is known to those skilled in the art.

Suitable pharmaceutically acceptable salts include, for example, chloride, sulfate, phosphate, citrate and oxylate.

### DEFINITIONS AND CONVENTIONS

The definitions and explanations below are for the terms as used throughout this entire document including both the specification and the claims.

### I. CONVENTIONS FOR FORMULAS AND DEFINITIONS OF VARIABLES

The chemical formulas representing various compounds or molecular fragments in the specification and claims may contain variable substituents in addition to expressly defined structural features. These variable substituents are identified by a letter or a letter followed by a numerical subscript, for example, "Z₁" or "Rᵢ" where "i" is an integer. These variable substituents are either monovalent or bivalent, that is, they represent a group attached to the formula by one or two chemical bonds. For example, a group Z₁ would represent a bivalent variable if attached to the formula CH₃-C(=Z₁)H. Groups Rᵢ and Rⱼ would represent monovalent variable substituents if attached to the formula CH₃-CH₂-C(Rᵢ)(Rⱼ)H₂. When chemical formulas are drawn in a linear fashion, such as those above, variable substituents contained in parentheses are bonded to the atom immediately to the left of the variable substituent enclosed in parenthesis. When two or more consecutive variable substituents are enclosed in parentheses, each of the consecutive variable substituents is bonded to the immediately preceding atom to the left which is not enclosed in parentheses. Thus, in the formula above, both Rᵢ and Rⱼ are bonded to the preceding carbon atom.

Chemical formulas or portions thereof drawn in a linear fashion represent atoms in a linear chain. The symbol "-" in general represents a bond between two atoms in the chain. Thus CH₃-O-CH₂-CH(Rᵢ)-CH₃ represents a 2-substituted-1-methoxypropane compound. In a similar fashion, the symbol "=" represents a double bond, e.g., CH₂-C(Rᵢ)-O-CH₃, and the symbol "≡" represents a triple bond, e,g., HC≡C-CH(Rᵢ)-CH₂-CH₃. Carbonyl groups are represented in either one of two ways: -CO- or -C(=O)-, with the former being preferred for simplicity.

Chemical formulas of cyclic (ring) compounds or molecular fragments can be represented in a linear fashion. Thus, the compound 4-chloro-2-methylpyridine can be represented in linear fashion by N^{*}-C(CH₃)-CH=CCl-CH-C^{*}H with the convention that the atoms marked with an asterisk (^{*}) are bonded to each other resulting in the formation of a ring. Likewise, the cyclic molecular fragment, 4-(ethyl)-1-piperazinyl can be represented by -N*-(CH₂)₂-N(C₂H₅)-CH₂-C^{*}H₂.

A rigid cyclic (ring) structure for any compounds herein defines an orientation with respect to the plane of the ring for substituents attached to each carbon atom of the rigid cyclic compound. For saturated compounds which have two substituents attached to a carbon atom which is part of a cyclic system, -C(X₁)(X₂)- the two substituents may be in either an axial or equatorial position relative to the ring and may change between axial/equatorial. However, the position of the two substituents relative to the ring and each other remains fixed. While either substituent at times may lie in the plane of the ring (equatorial) rather than above or below the plane (axial), one substituent is always above the other. In formulas depicting such compounds, a substituent (X₁) which is "below" another substituent (X₂) will be identified as being in the alpha (a) configuration and is identified by a broken, dashed or dotted line attachment to the carbon atom, i.e., by the symbol "- - -" or "...". The corresponding substituent attached "above" (X₂) the other (X₁) is identified as being in the beta (β) configuration and is indicated by an unbroken line attachment to the carbon atom.

When a variable substituent is bivalent, the valences may be taken together or separately or both in the definition of the variable. For example, a variable Rᵢ attached to a carbon atom as -C(-Rᵢ)- might be bivalent and be defined as oxo or keto (thus forming a carbonyl group (-CO-) or as two separately attached monovalent variable substituents α-Rᵢ₋ⱼ and β-Rᵢ₋ₖ. When a bivalent variable, Rᵢ, is defined to consist of two monovalent variable substituents, the convention used to define the bivalent variable is of the form "α-Rᵢ₋ⱼ:β-Rᵢ₋ₖ" or some variant thereof. In such a case both α-Rᵢ₋ⱼ and β-Rᵢ₋ₖ are attached to the carbon atom to give -C(α-Rᵢ₋ⱼ)(β-Rᵢ₋ₖ)-. For example, when the bivalent variable R₆, -C(-R₆)- is defined to consist of two monovalent variable substituents, two monovalent variable substituents are α-R₆₋₁:β-R₆₋₂, .... α-R₆₋₉:β-R₆₋₁₀, etc, giving -C(α-R₆₋₁)(β-R₆₋₂)-, .... -C(α-R₆₋₉) (β-R₆₋₁₀)-, etc. Likewise, for the bivalent variable R₁₁, -C(=R₁₁)-, two monovalent variable substituents are α-R₁₁₋₁:β-R₁₁₋₂. For a ring substituent for which separate α and β orientations do not exist (e.g. due to the presence of a carbon carbon double bond in the ring), and for a substituent bonded to a carbon atom which is not part of a ring the above convention is still used, but the α and β designations are omitted.

Just as a bivalent variable may be defined as two separate monovalent variable substituents, two separate monovalent variable substituents may be defined to be taken together to form a bivalent variable. For example, in the formula -C₁(Rᵢ)H-C₂(Rⱼ)H- (C₁ and C₂ define arbitrarily a first and second carbon atom, respectively) Rᵢ and Rⱼ may be defined to be taken together to form (1) a second bond between C₁ and C₂ or (2) a bivalent group such as oxa (-0-) and the formula thereby describes an epoxide. When Rᵢ and Rⱼ are taken together to form a more complex entity, such as the group -X-Y-, then the orientation of the entity is such that C₁ in the above formula is bonded to X and C₂ is bonded to Y. Thus, by convention the designation "... Rᵢ and Rⱼ are taken together to form -CH₂-CH₂-O-CO- ..." means a lactone in which the carbonyl is bonded to C₂. However, when designated "... Rⱼ and Rᵢ are taken together to form -CH₂-CH₂-O-CO- the convention means a lactone in which the carbonyl is bonded to C₁.

The carbon atom content of variable substituents is indicated in one of two ways. The first method uses a prefix to the entire name of the variable such as "C₁-C₄", where both "1" and "4" are integers representing the minimum and maximum number of carbon atoms in the variable. The prefix is separated from the variable by a space. For example, "C₁-C₄ alkyl" represents alkyl of 1 through 4 carbon atoms, (including isomeric forms thereof unless an express indication to the contrary is given). Whenever this single prefix is given, the prefix indicates the entire carbon atom content of the variable being defined. Thus C₂-C₄ alkoxycarbonyl describes a group CH₃-(CH₂)ₙ-O-CO- where n is zero, one or 2. By the second method the carbon atom content of only each portion of the definition is indicated separately by enclosing the "Cᵢ-Cⱼ" designation in parentheses and placing it immediately (no intervening space) before the portion of the definition being defined. By this optional convention (C₁-C₃)alkoxycarbonyl has the same meaning as C₂-C₄ alkoxycarbonyl because the "C₁-C₃" refers only to the carbon atom content of the alkoxy group. Similarly while both C₂-C₆ alkoxyalkyl and (C₁-C₃)alkoxy(C₁-C₃)alkyl define alkoxyalkyl groups containing from 2 to 6 carbon atoms, the two definitions differ since the former definition allows either the alkoxy or alkyl portion alone to contain 4 or 5 carbon atoms while the latter definition limits either of these groups to 3 carbon atoms.

### II. DEFINITIONS

All temperatures are in degrees Centigrade.

TLC refers to thin-layer chromatography,

THF refers to tetrahydrofuran.

THP refers to tetrohydropyanyl.

DMF refers to dimethylformamide.

TEA refers to triethylamine.

Alcohol refers to ethyl alcohol.

MPLC refers to medium pressure liquid chromatography,

Saline refers to an aqueous saturated sodium chloride solution.

IR refers to infrared spectroscopy.

CMR refers to C-13 magnetic resonance spectroscopy, chemical shifts are reported in ppm (δ) downfield from TMS.

NMR refers to nuclear (proton) magnetic resonance spectroscopy, chemical shifts are reported in ppm (δ) downfield from tetramethylsilane.

TMS refers to trimethylsilyl.

φ refers to phenyl (C₆H₅).

MS refers to mass spectrometry expressed as m/e or mass/charge unit. [M + H]⁺ refers to the positive ion of a parent plus a hydrogen atom. EI refers to electron impact. CI refers to chemical ionization. FAB refers to fast atom bombardment.

Pharmaceutically acceptable refers to those properties and/or substances which are acceptable to the patient from a pharmacological/toxicological point of view and to the manufacturing pharmaceutical chemist from a physical/chemical point of view regarding composition, formulation, stability, patient acceptance and bioavailability.

When solvent pairs are used, the ratios of solvents used are volume/volume (v/v).

_{∼} indicates that there are 2 possible orientations for the attached group, (1) α or β when attached to the ring and (2) cis or trans when attached to a carbon atom of a double bond.

BOC refers to t-butyloxycarbonyl, -CO-O-C(CH₃)₃.

CBZ refers to carbobenzyloxy, -CO-O-CH₂-φ.

### EXAMPLES

Without further elaboration, it is believed that one skilled in the art can, using the preceding description, practice the present invention to its fullest extent. The following detailed examples describe how to prepare the various compounds and/or perform the various processes of the invention and are to be construed as merely illustrative, and not limitations of the preceding disclosure in any way whatsoever. Those skilled in the art will promptly recognize appropriate variations from the procedures both as to reactants and as to reaction conditions and techniques.

### EXAMPLE 1 1-Carbo-t-butyloxy-5-nitroindazole (XXIII)

A mixture of 5-nitroindazole (XXII, 5.685) and di-t-butyl dicarbonate (15.325 g) is stirred for four days in refluxing THF (freshly distilled, 220 ml) under nitrogen. The mixture is then concentrated to 70 ml, by distillation, and then poured over crushed ice (600 ml). After the ice melts, the mixture is filtered using reduced pressure. The precipitate is dried in a vacuum oven to give a solid. A small amount (536 mg) of this product is purified by passing it through a silica gel column (23.5 x 2.5 cm, 40-63 *µ*) eluting with ethyl acetate/hexane (1/3, 700 ml and then 1/1 200 ml). The appropriate fractions are pooled and concentrated to a solid. The solid is recrystallized from acetone to give the title compound, NMR (CDCl₃, 300 MHz) 8.71, 8.43-8.32 and 1.76 δ; CMR (CDCl₃, 75.47 MHz) 27.91, 86.19, 114.956, 117.82, 123.54, 125.27, 140.06, 141.79, 144.08 and 148.30 δ; IR (mineral oil mull) 1765, 1736, 1532, 1383, 1347, 1291, 1155 and 1151 cm⁻¹; MS (m/e) 263, 204, 163, 57 and 40.

### EXAMPLE 2 1-Carbo-t-butyloxy-5-aminoindazole (XXIV)

To a solution of 1-carbo-t-butyloxy-5-nitroindazole (XXIII, EXAMPLE 1, 6.165 g) in ethyl acetate (125 ml) is added palladium on carbon (10%, 734 mg). The mixture is stirred under hydrogen (1 atm. balloon) at 20-25°. After stirring for 27 hours, more palladium on carbon (10%, 294 mg) is added. Then, after stirring for an additional two days under hydrogen, the mixture is filtered over dicalite and the filtrate is concentrated to an oil. The oil is taken up in ethyl acetate (125 ml), dried over magnesium sulfate and concentrated to an oil. The oil is passed over a silica column (27 x 4.5 cm, 40-63 µ) eluting with ethyl acetate/hexane (1/3, 500 ml followed by 1/1 1000 ml), ethyl acetate (1000 ml) and methanol/ethyl acetate (1/9, 1000 ml). The appropriate fractions are pooled and concentrated to give the title compound, NMR (CDCl₃, 300 MHz) 7.99-7.94, 6.97-6.91, 3.77 and 1.71 δ; CMR (CDCl₃, 75.47 MHz) 28.06, 84.335, 103.82, 115.05, 119.35, 126.84, 134.07, 138.68, 142.64 and 149.13 δ; IR (mineral oil mull) 1736, 1518, 1462, 1375, 1301, 1232 and 1145 cm^{-1;} MS (m/e) 233, 133, 105, 57 and 40.

### EXAMPLE 3 1-Carbo-t-butyloxy-5-(N-carbobenzyloxy)aminoindazole (XXV)

Benzyl chloroformate (2.45 ml) is added td a mixture of 1-carbot-butyloxy-5-aminoindazole (XXIV, EXAMPLE 2, 3.760 g) and sodium bicarbonate (2.721 g) in acetone/water (1/1, 50 ml) at 0° over one minute. The mixture is stirred under nitrogen for 1.5 hr then poured into water (50 ml). The aqueous mixture is then extracted with ethyl acetate (3 x, 250 ml total). The combined organic layers are washed with aqueous sodium bisulfate (10%, 125 ml), aqueous sodium bicarbonate (10%, 125 ml), saline (125 ml), dried over magnesium sulfate, and concentrated to a give the title compound as a solid, NMR (CDCl₃, 300 MHz) 8.08-7.98, 7.48-7.27, 5.20 and 1.70 δ; CMR (CDCl₃, 75.47 MHz) 28.03, 66.99, 84.82, 109.7, 114.77, 121.1, 126.19, 128.19, 128.26, 128.50, 133.93, 135.84, 136.20, 139.32, 149.00 and 153.59 δ; IR (mineral oil mull) 1746, 1726, 1521, 1395, 1355, 1290, 1218 and 1044 cm⁻¹; MS (m/e) 367, 267, 223, 132, 91, 57 and 40.

### EXAMPLE 4 1-Carbo-t-butyloxy-5-(N-allyl-N-carbobenzyloxy)aminoindazole (XXVI) 1-Allyl-5-(N-allyl-N-carbobenzyloxy)aminoindazole (XXVI')

Allyl bromide (1.70 ml) is added to a mixture of 1-carbo-t-butyloxy-5-(N-carbobenzyloxy)aminoindazole (XXV, EXAMPLE 3, 5.805 g) and sodium hydride/mineral oil (50% by weight, 1.000 g, 15.8 mmol sodium hydride) in freshly distilled THF (80 ml). The mixture is refluxed for 20 hr under nitrogen, then poured into water (100 ml). The aqueous mixture is extracted with ethyl acetate (3 x 100 ml). The combined organic layers are washed with saline, dried over magnesium sulfate, and concentrated to an oil. The oil is passed over a silica gel column (26 x 4.5 cm, 40-63 µ), eluting with ethyl acetate/hexane (1/4 2 1, 1/1 1 1) and ethyl acetate (300 ml) collecting 47 ml fractions. The appropriate fractions are pooled and concentrated give l-carbo-t-butyloxy-5-(N-allyl-N-carbobenzyloxy)aminoindazole (XXVI) NMR (CDCl₃, 300 MHz) 8.14, 7.59, 7.34, 5.93, 5.16, 5.11, 4.32 and 1.71 δ; CMR (CDCl₃, 75.47 MHz) 27.99, 53.53, 67.32, 84.91, 114.51, 114.75, 126.00, 127.56, 127.85, 128.30, 132.67, 133.294, 137.93, 139.24, 148.87 and 155.24 δ; MS (m/e) 407, 307, 172, 91, 57 and 40. Later eluting fractions are pooled and concentrated give 1-allyl-5-(N-allyl-N-carbobenzyloxy)aminoindazole (XXVI') NMR (CDCl₃, 300 MHz) 7.91, 7.68, 7.47, 7.28, 7.14, 6.18-6.05, 6.00-5.87, 5.37-5.29, 5.16-5.11, 5.04-5.01 and 4.29 δ; CMR (CDCl₃, 75.47 MHz, major peaks) 53.59, 55.91, 67.08, 117.20, 117.56, 117.93, 119.28, 121.48, 123.12, 126.72, 127.43, 127.70, 128.25, 132.07, 133.65, 136.55, 147.39 and 155.46 δ.

### EXAMPLE 5 (±)-3-[5'-(1-Carbo-t-butyloxyindazolyl)]-5-(iodomethyl)oxazolidin-2-one (XXVII) (±)-3-[5'-(1-Allylindazolyl)]-5-(iodomethyl)oxazolidin-2-one (XXVII')

Iodine (4.699 g) is added to 3.580 g of a mixture of 1-carbo-t-butyloxy-5-(N-allyl-N-carbobenzyloxy)aminoindazole (XXVI, EXAMPLE 4) and 1-allyl-5-(N-allyl-N-carbobenzyloxy)aminoindazole (XXVI', EXAMPLE 4) in chloroform (95 ml). The mixture is stirred under nitrogen for 1.5 hr then poured into aqueous sodium thiosulfate (10%, 100 ml). The layers are separated, and the organic layer is washed with additional aqueous sodium thiosulfate (10%, 2 x 50 ml). The aqueous layers are combined and extracted with ethyl acetate (3 x, 200 ml total). The organic layers are combined, dried over magnesium sulfate and concentrated to give an oil. The oil is adsorbed onto silica gel (40-63 µ) then placed on a silica gel column (35 x 5.5 cm, 40-63 µ) eluting with ethyl acetate/:hexane (1/3, 500 ml; 1/1, 2 1) and methanol/ethyl acetate (1/9, 2 1) collecting 41 ml fractions. The appropriate fractions are pooled and concentrated give (±)-3-[5'(1-allylindazolyl)]-5-(iodomethyl)oxazolidin-2-one (XXVII'), NMR (CDCl₃, 300 MHz) 7.98, 7.72, 7.68, 7.4, 6.01, 5.22, 5.14, 5.08, 5.02, 5.00, 4.73, 4.23, 3.84, 3.47 and 3.39 δ; CMR (CDCl₃, 75.47 MHz) 6.29, 51.76, 51.87, 71.09, 109.86, 110.86, 117.76, 119.68, 123.97, 131.30, 132.45, 132.98, 136.90 and 154.4; IR (Neat) 1746, 1510, 1417, 1226 and 1112 cm⁻¹; MS (m/e) 383, 255, 212, 184, 170, 157 and 40.

Later eluting fractions are pooled and concentrated give (±)-3-[5'-(1-carbo-t-butyloxyindazolyl)]-5-(iodomethyl)oxazolidin-2-one (XXVII) which is recrystallized from acetone, NMR (CDCl₃, 300 MHz) 8.18, 7.87, 7.78, 4.78, 4.27, 3.88, 3.51, 3.41 and 1.73 δ; CMR (CDCl₃, 75.47 MHz) 5.95, 28.04, 51.42, 71.14, 85.04, 110.16, 115.01, 120.51, 125.98, 133.85, 136.8, 139.22, 149.1 and 154.5 δ; IR (mineral oil mull) 1745, 1390 and 1155 cm⁻¹; MS (m/e) 443, 343, 172, 144, 117, 57 and 40.

### EXAMPLE 6 (±)-3-(5'-Indazolyl)-5-(azidomethyl)oxazolidin-2-one (XXVIII) (±)-3-[5'-(1-Allylindazolyl)]-5-(azidomethyl)oxazolidin-2-one (XXVIII')

A mixture (2.515 g) of (±)-3-[5'-(1-carbo-t-butyloxyindazolyl)]5-(iodomethyl)oxazolidin-2-one (XXVII, EXAMPLE 5) and (±)-3-[5'-(1-allylindazolyl)]-5-(iodomethyl)oxazolidin-2-one (XXVII', EXAMPLE 5) is stirred with sodium azide (2.575 g) in refluxing water/acetone (1/2, 150 ml) under nitrogen for 25 hr. The mixture is then poured into ethyl acetate (100 ml). The layers are separated. The aqueous phase is extracted with ethyl acetate (3 x 25 ml). The combined organic layers are dried over magnesium sulfate and concentrated to an oil. A sample of the oil is purified by preparative TLC. From the purification (±)-3-(5'-indazolyl)-5-(azidomethyl)oxazolidin-2-one (XXVIII), NMR (CDCl₃ ca. 0.5% DMF-d₇, 300 MHz) 9.5-8.5, 8.02, 7.69, 7.55, 4.84, 4.20, 3.92, 3.74 and 3.62 δ; CMR (CDCl₃, ca. 0.5% DMF-d₇, 75.47 MHz, 300 MHz) 48.24, 52.96, 70.66, 110.38, 110.75, 122.8, 131,5, 133.9, 137.8 and 154.9 δ; IR (neat) 2108, 1741, 1511, 1420 and 1277 and (±)-3-[5'-(1-allylindazolyl)]-5-(azidomethyl)oxazolidin-2-one (XXVIII') NMR (CDCl₃, 300 MHz) 7.99, 7.74, 7.68, 7.42, 6.02, 5.23, 5.12, 5.02, 4.82, 4.16, 3.94, 3.73 and 3.62 δ; CMR (CDCl₃, 75.47 MHz) 48.33, 51.78, 52.98, 70.54, 109.88, 110.79, 117.76, 119.65, 124.1, 131.7, 132.42, 132.98, 137.3 and 154.6 δ; IR (neat) 2105, 1746, 1510, 1418 and 1224 cm⁻¹.

### EXAMPLE 7 (±)-3-(5'-Indazolyl)-5-(aminomethyl)oxazolidin-2-one (XXIX) (±)-3-[5'-(1-n-Propylindazolyl)]-5-(aminomethyl)oxazolidin-2-one (XXIX')

Palladium on carbon (10%, 540 mg) is added to a combined mixture (2.000 g) of (±)-3-(5'-indazolyl)-5-(azidomethyl)oxazolidin-2-one (XXVIII, EXAMPLE 6) and (±)-3-[5'-(1-allylindazolyl)]-5-(azidomethyl)oxazolidin-2-one (XXVIII', EXAMPLE 6) in methanol/ethyl acetate (105, 110 ml). The mixture is stirred under 1 atm of hydrogen (balloon) overnight. The mixture is then filtered over diatomaceous earth and the filtrate concentrated to a tar. The crude material is dissolved and is passed over a silica gel column (23 x 4 cm, 40-63 *µ*) eluting with methanol/chloroform (1/9, 600 ml, 1/4, 1.5 1) collecting 46 ml fractions. The appropriate fractions are pooled and concentrated give (±)-3-[5'-(1-n-propylindazolyl)]-5-(aminomethyl)oxazolidin-2-one (XXIX'), NMR (MeOD, 300 MHz) 7.96, 7.73, 7.52, 4.71, 4.31, 4.14, 3.87, 2.98, 1.87 and 0.84 δ; CMR (MeOD, 75.47 MHz) 11.64, 24.35, 45.61, 50.12, 51.37, 75.62, 110.96, 112.42, 121.60, 124.95, 133.32, 133.81, 138.50 and 157.50 δ; IR (neat) 1741, 1510, 1418, 1225 and 1113 cm⁻¹. Later eluting fractions are pooled and concentrated give (±)-3-(5'-indazolyl)-5-(aminomethyl)oxazolidin-2-onee (XXIX), NMR (MeOD, 300 MHz) 8.02, 7.77, 7.70, 7.55, 4.78, 4.17, 3.89 and 3.05 δ; CMR (MeOD, 75.47 MHz) 45.43, 50.35, 75.35, 111.76, 112.52, 122.02, 124.19, 133.16, 134.99, 139.20 and 157.60 δ; IR (neat) 3800-3000 very broad, 1735, 1511 and 1423 cm⁻¹.

### EXAMPLE 8 (±)-3-[5'-(1-Acetylindazolyl)]-5-(acetamidomethyl)oxazolidin-2-one (XXX) (±)-3-(5'-Indazolyl)-5-(acetamidomethyl)oxazolidin-2-one (XXXI)

Acetic anhydride (0.5 ml) is added to (±)-3-(5'-indazolyl)-5-(aminomethyl)oxazolidin-2-one (XXIX, EXAMPLE 7, 152 mg) in pyridine (1,5 ml) at 0°. The mixture is stirred for two hr while allowing it to warm to 20-25°. The mixture is then concentrated under reduced pressure to a solid. The solid is purified on a silica preparative plate (1000 µ) developing with methanol/chloroform (1/10) to give (±)-3-(5'-(1-acetylindazolyl))-5-acetamidomethyl-2-oxazolidin-2-one (XXX), NMR (CH₃OD, 300 MHz) 8.03, 7.76, 7.70, 7.54, 4.79, 4.20, 3.89, 3.58 and 1.98 δ; CMR (CH₃OD, 75.47 MHz) 22.49, 43.23, 50.28, 73.58, 111.70, 112.43, 124,.3, 133.5, 135.2, 139.6, 157.9 and 174.5 δ and (±)-3-(5'-indazolyl)-5-acetamidomethyl-2-oxazolidin-2-one (XXXI), NMR (CDCl₃, 300 MHz) 8.37, 8.08, 7.85, 7.69, 6.61, 4.83, 4.14, 3.91, 3.70, 2.78 and 2.04 δ; CMR (CDCl₃, 75.47 MHz) 22.73, 22.93, 41.77, 47.94, 71.95, 109.92, 115.82, 120.64, 126.51, 134.55, 135.6, 139.37, 154.5, 170.75 and 171.16 δ.

### EXAMPLE 9 (±)-3-[5'-(1-Ethylindazolyl)]-5-(acetamidomethyl)oxazolidin-2-one (XXXII)

Starting with (±)-3-(5'-indazolyl)-5-(acetamidomethyl)oxazolidin-2-one (XXXI, EXAMPLE 8) in methanol and acetaldehyde (2 equivalents), the mixture is treated with glacial acetic acid to bring the pH to 5. After stirring the mixture for 1-2 hr, 1 equivalent of sodium cyanoborohydride is added and the mixture is stirred for 24 hr at 20-25^{*}. The mixture is then concentrated under reduced pressue. Water is added, and the pH is adjusted to 7-8 with 1N aqueous potassium hydroxide, then extracted with chloroform (3x). The organic extracts are combined, dried over magnesium sulfate and concentrated under reduced pressure to give the title compound. It can be purified by recrystallization or column chromatography on silica gel if desired.

### EXAMPLE 10 (±)-3-[5'-(1-n-Propylindazolyl)]-5-(acetamidomethyl)oxazolidin-2-one (XXX')

Acetic anhydride (0.5 ml) is added to (±)-3-[5'-(1-n-propylindazolyl)]-5-(aminomethyl)oxazolidin-2-one (XXIX', EXAMPLE 7, 126 mg) in pyridine (1.5 ml) at 0°. The mixture is stirred for two hr while allowing it to warm to 20-25°. The mixture is then concentrated under reduced pressure to a solid. The solid is purified on a silica preparative plate (1000 µ) developing with with methanol/chloroform (1/10) to give the title compound, NMR (CDCl₃, 300 MHz) 7.95, 7.66, 7.39, 6.91, 4.80, 4.33, 4.10, 3.89, 3.66, 2.02, 1.93 and 0.90 δ; CMR (CDCl₃, 75.47 MHz): δ 11.22, 22.84, 23.09, 41.81, 48.53, 50.53, 71.98, 109.51, 110.98, 119.53, 123.62, 131.11, 132.47, 136.92, 155.19 and 171.27 δ.

### EXAMPLE 11 3-(5'-Indazolyl)-5β-(aminomethyl)oxazolidin-2-one (XXIX)

(±)-3-(5'-Indazolyl)-5-(aminomethyl)oxazolidin-2-one (XXIX, EXAMPLE 7) is stirred with (+) or (-) tartaric acid in methylene chloride and then permitted to stand while the product crystallizes out. The crystalline product is obtained by filtration and treated with triethylamine or sodium bicarbonate to obtain the free amine which is obtained by extraction with methylene chloride. The methylene chloride extract is concentrated to give the title compound.

### EXAMPLE 12

Following the general procedure of EXAMPLE 11 and making noncritical variations but starting with the racemic mixture of EXAMPLE 7, the following compound is obtained:
3-[5'-(1-n-Propylindazolyl)]-5β-(aminomethyl)oxazolidin-2-one (XXIX')

### EXAMPLE 13 3-[5'-(1-Acetylindazolyl)]-5β-(acetamidomethyl)oxazolidin-2-one (XXX) 3-(5'-Indazolyl)-5β-(acetamidomethyl)oxazolidin-2-one (XXXI)

Following the general procedure of EXAMPLE 8 and making noncritical variations but starting with the optically active 3-(5'-indazolyl)-5β-(aminomethyl)oxazolidin-2-one (XXIX, EXAMPLE 11) the title compounds are obtained.

### EXAMPLE 14 3-[5'-(1-Ethylindazolyl)]-5β-(acetamidomethyl)oxazolidin-2-one (XXXII)

Following the general procedure of EXAMPLE 9 and making noncritical variations but starting with the optically active 3-(5'-indazolyl)-5β-(acetamidomethyl)oxazolidin-2-one (XXXI, EXAMPLE 13) the title compound is obtained.

### EXAMPLE 15 3-[5'-(1-n-Propylindazolyl)]-5β-(acetamidomethyl)oxazolidin-2-one (XXX')

Following the general procedure of EXAMPLE 10 and making noncritical variations but starting with the optically active 3-[5'-(1-n-propylindazolyl)]-5β-(aminomethyl)oxazolidin-2-one (XXIX', EXAMPLE 12) the title compound is obtained.

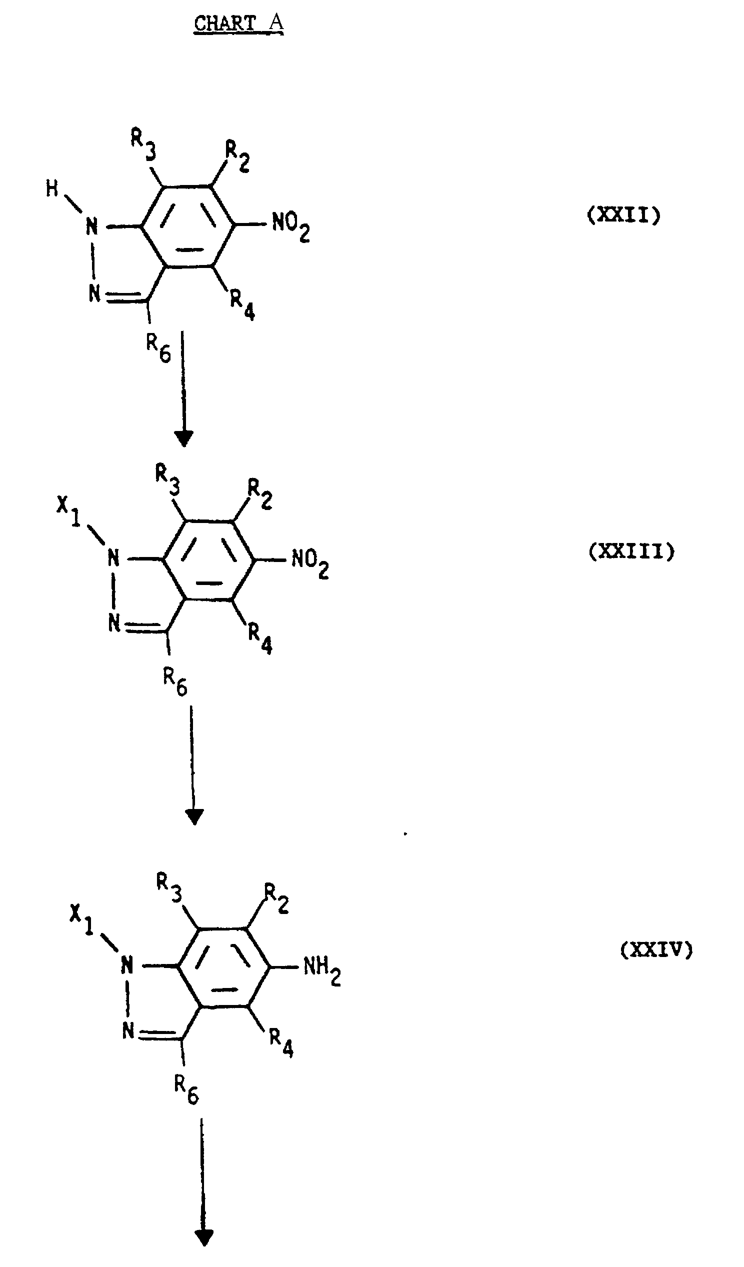

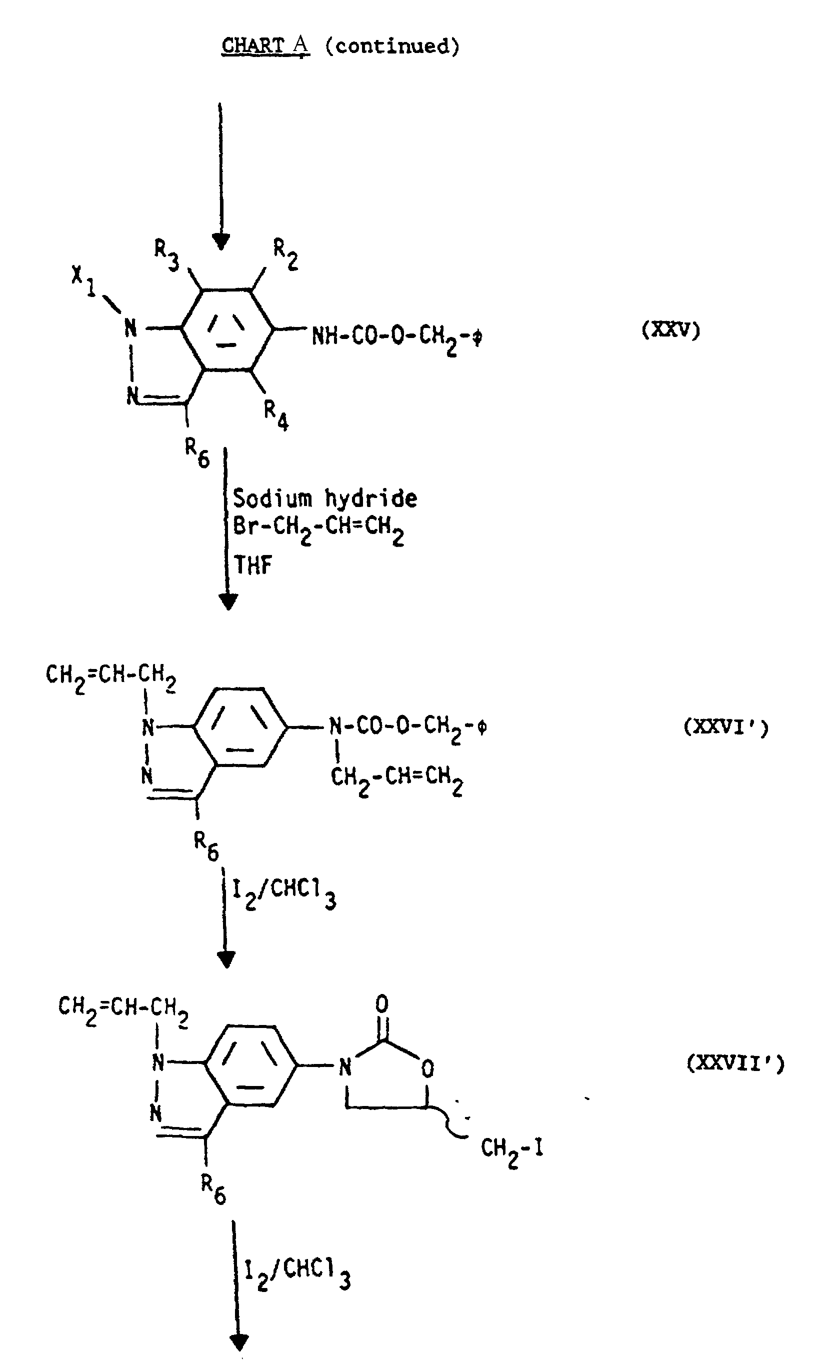

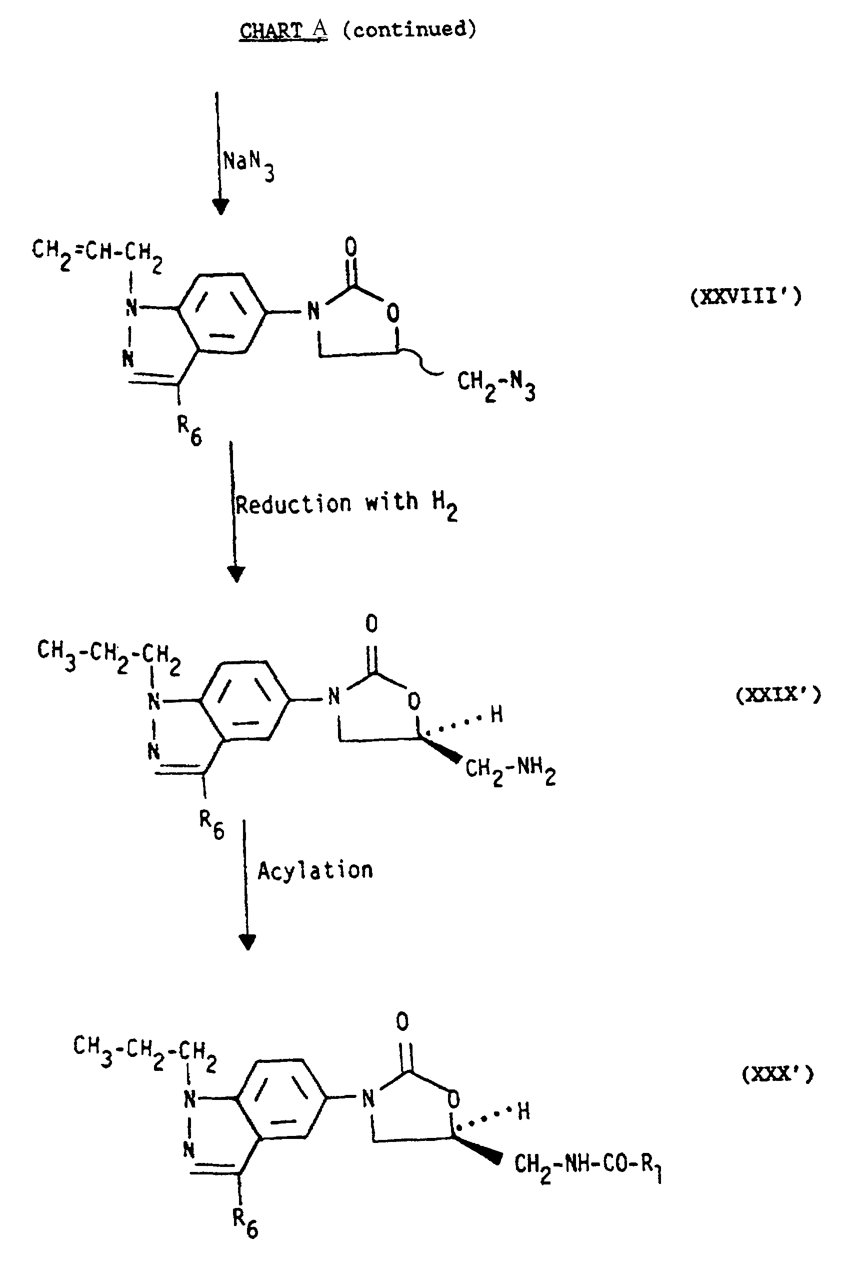

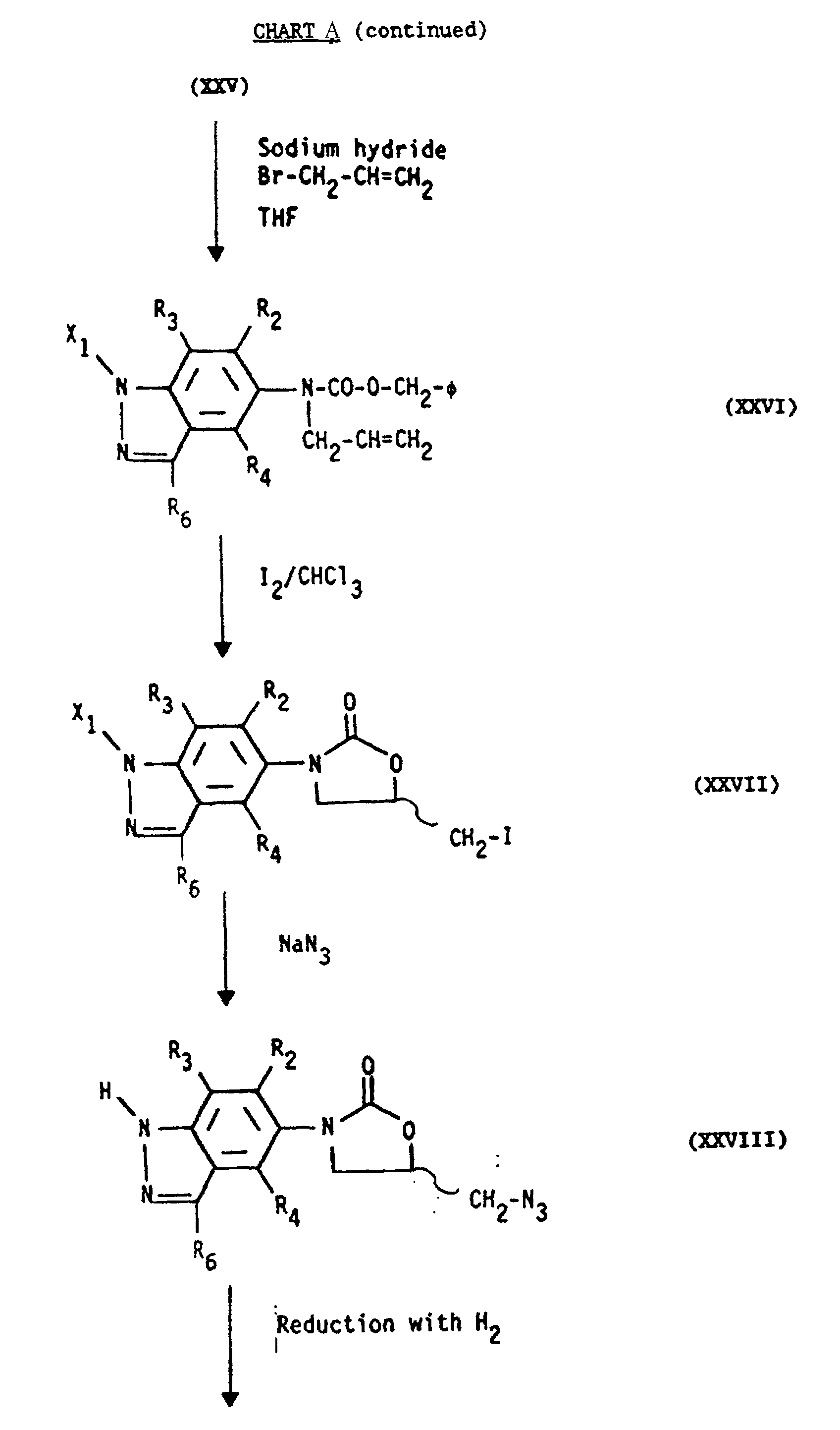

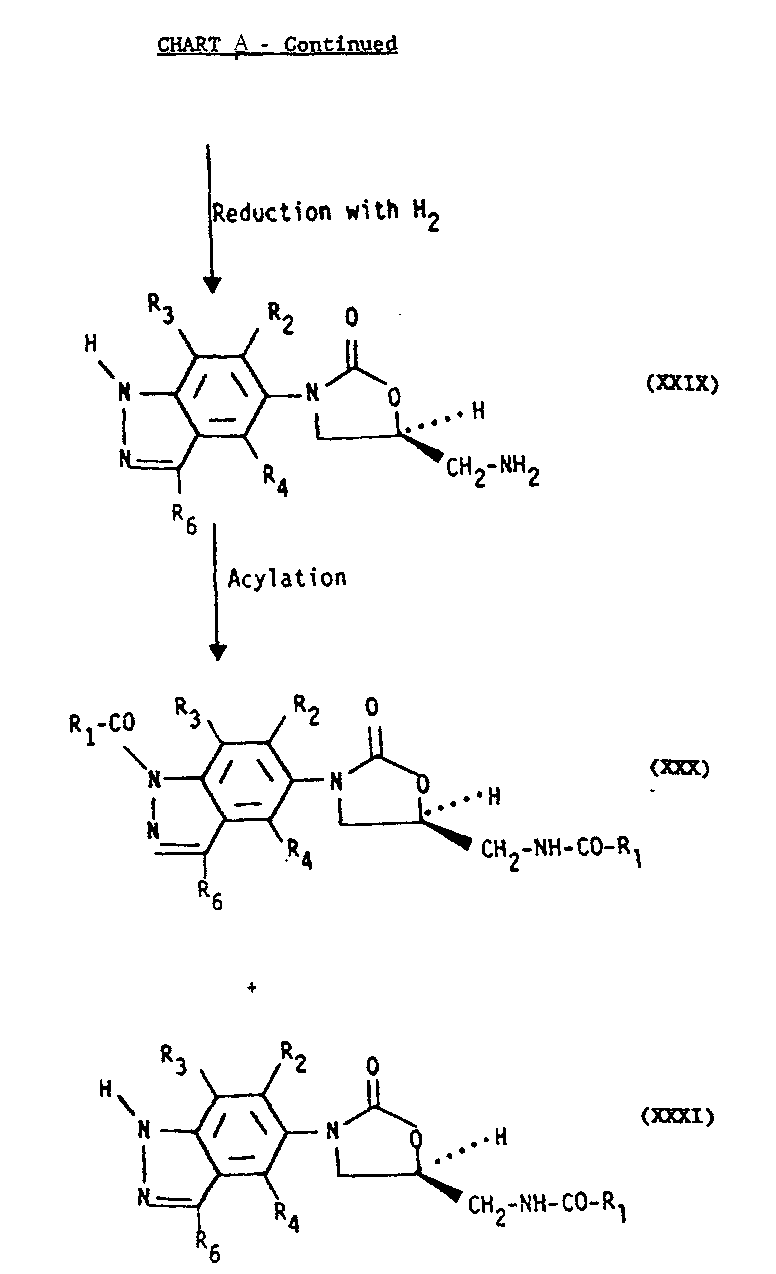

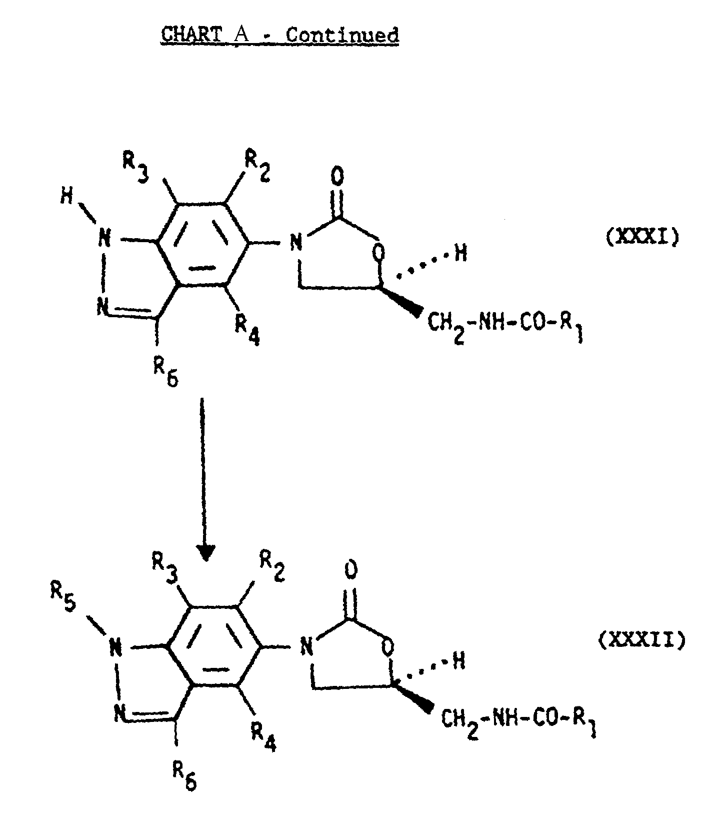

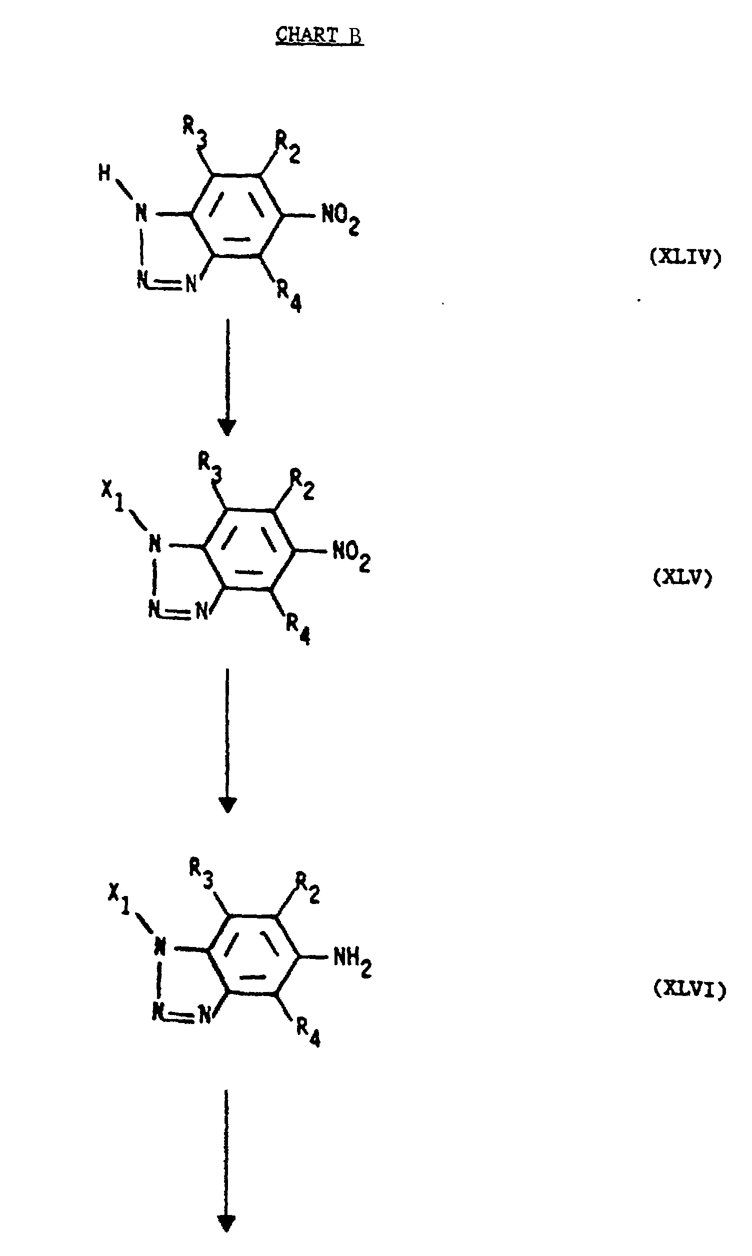

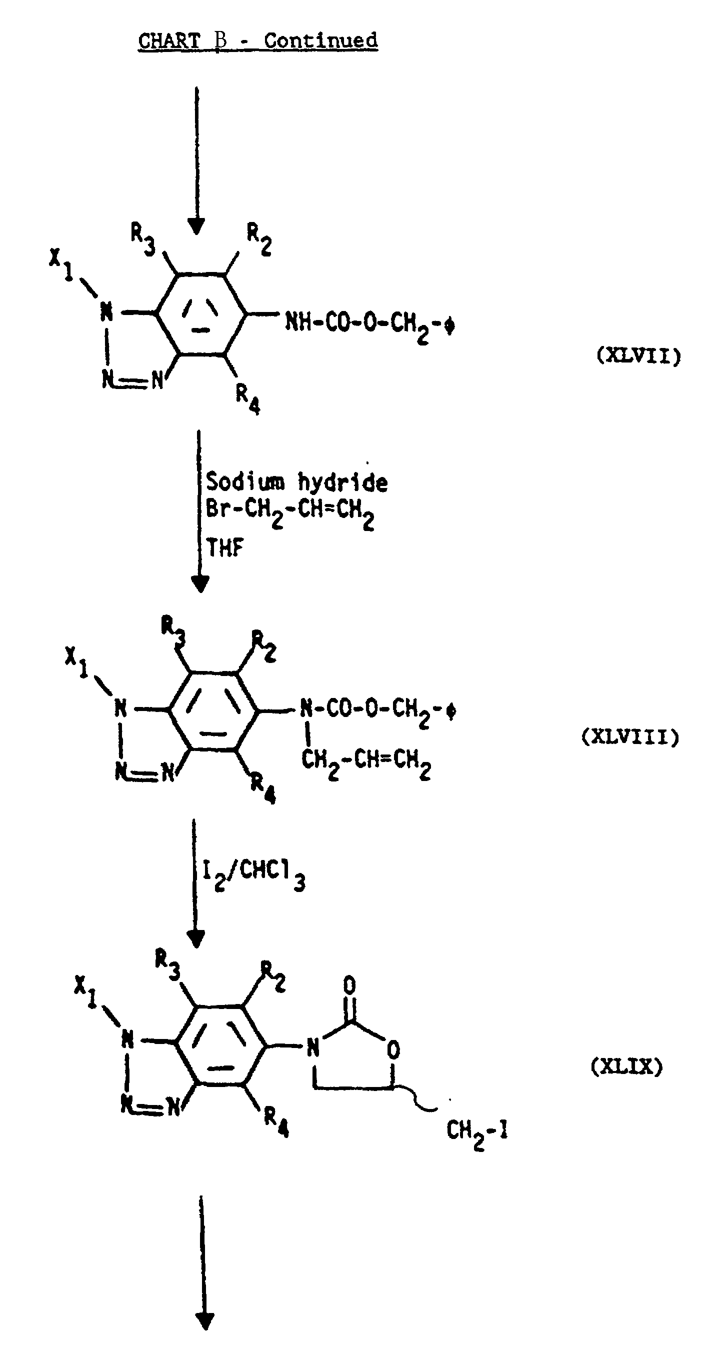

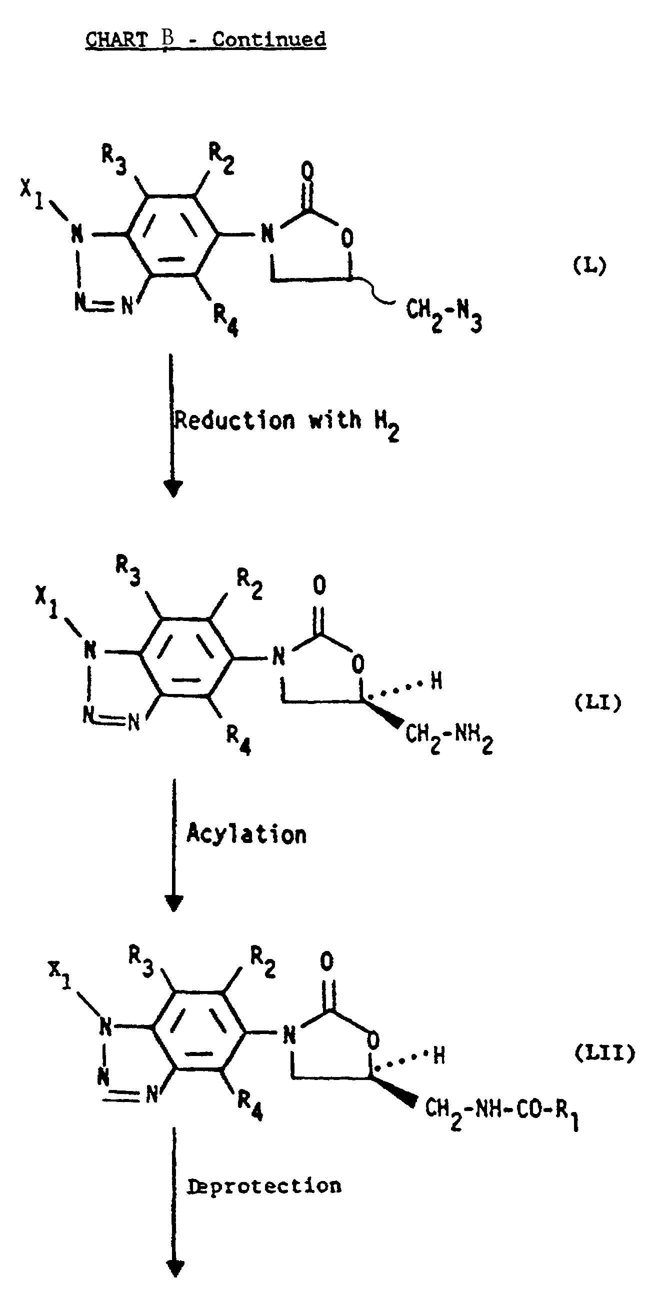

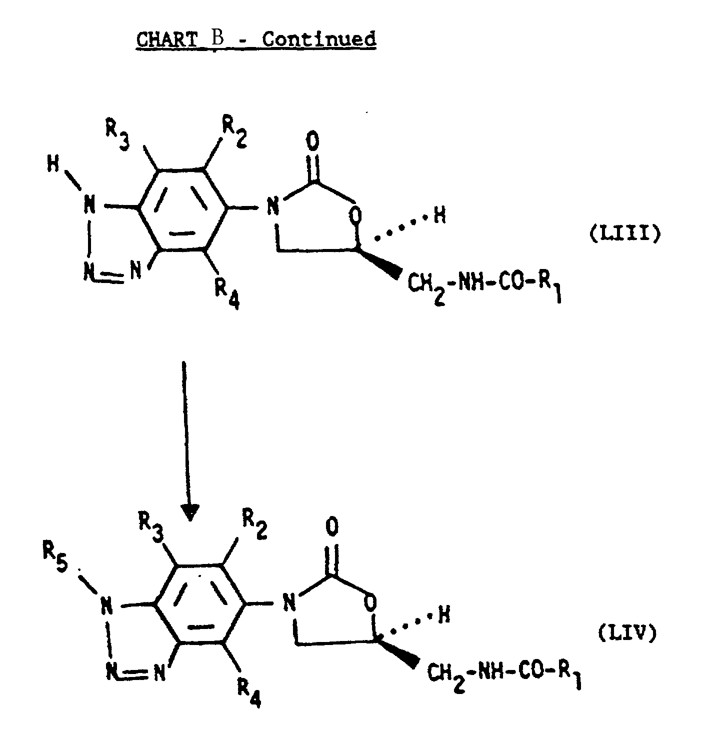

## Claims

1. A 3-(nitrogen substituted)phenyl-5β-(amidomethyl)oxazolidin-2-one of formula (LV) 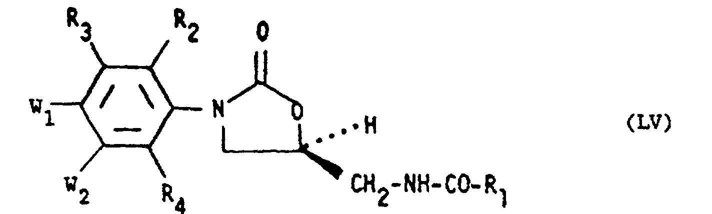 where
(I) R₁ is -H,
C₁-C₁₂ alkyl optionally substituted with 1-3 Cl,
C₃-C₁₂ cycloalkyl,
C₅-C₁₂ alkenyl containing one double bond,
-φ optionally substituted with 1-3 groups selected from -OH, -OCH₃, -OC₂H₅, -NO₂, -F, -Cl, -Br, -COOH, -SO₃H and -N(R₁₋₁)(R₁₋₂) where R₁₋₁ and R₁₋₂ are the same or different and are -H or C₁-C₂ alkyl,
furanyl,
tetrahydrofuranyl,
2-thiophene,
pyrrolidinyl,
pyridinyl,
-O-R₁₋₃ where R₁₋₃ is C₁-C₄ alkyl,
-NH₂,
-NHR₁₋₃ where R₁₋₃ is as defined above,
-NR₁₋₃R₁₋₄ where R₁₋₄ is C₁-C₃ alkyl and R₁₋₃ is as defined above, and where R₁₋₃ and R₁₋₄ can be taken together with the attached nitrogen atom to form a saturated mono-nitrogen C₅-C₇ heterocyclic ring including -O-,
-CH₂-OH,
-CH₂-OR₁₋₅ where R₁₋₅ is C₁-C₄ alkyl or -CO-R₁₋₆ where R₁₋₆ is C₁-C₄ alkyl or -φ;
(II) two of R₂, R₃ and R₄ are -H and the other of R₂, R₃ and R₄ is -H,
-F, -Cl, Br, -I,
C₁-C₆ alkyl,
-OH,
-CO-O-R₂₋₁ where R₂₋₁ is C₁-C₄ alkyl or -φ,
-O-R₂₋₁ where R₂₋₁ is as defined above,
-COOH,
-COO⁻,
-CHO,
-CO-NR₂₋₂R₂₋₃ where R₂₋₂ and R₂₋₃ are the same or different and are -H, C₁-C₃ alkyl, or R₂₋₂ and R₂₋₃ are taken together with the attached nitrogen atom to form a saturated mono-nitrogen C₃-C₆ heterocyclic ring optionally containing -O-,
-C≡N,
-C≡CH,
-N≡C,
-CHOH-CH₃,
-CO-CH₃,
-SH,
-SO-CH₃,
-SO-φ,
-S-CH₃,
-S-φ,
-SO₂-CH₃,
-SO₂-φ,
-SO₃H,
-SO₂-NH₂,
-N₃,
-NO₂,
-NR_{2-4R2-5} where R₂₋₄ and R₂₋₅ are the same or different and are -H and C₁-C₃ alkyl,
-NH-CO-R₂₋₆ where R₂₋₆ is C₁-C₄ alkyl or -φ,
-NH-CO-NH₂,
-CH=CH₂,
-CH₂-CH=CH₂,
-CH=N-OH,
-CH=N-OCH₃, 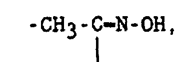 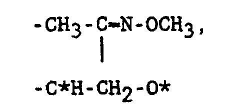 where the atoms marked with the asterisk (*) are bonded to each other resulting in the formation of a ring,
(IIIA) where W₁ and W₂ taken together are
-NR₅-N=CR₆- (XXXII)
where R₅ is
C₁-C₁₂ alkyl,
-CH₂-φ,
-CH₂CH₂-φ,
C₃-C₇ cycloalkyl,
C₂-C₁₂ alkenyl containing from 1 thru 3 double bonds,
C₂-C₁₂ alkynyl containing 1 triple bond,
-CHO,
-CO-R₅₋₁ where R₅₋₁ is
(A) C₁-C₆ alkyl optionally substituted with 1 -O-CH₃, -COOH, -NH₂, -SO₃H or 1-3 -Cl,
(B) C₃-C₇ cycloalkyl,
(C) 2-pyridinyl, 2-thiophene, 2-thiophenemethyl or 2-pyrrole,
(D) -φ optionally substituted with 1-3
-F, -Cl, Br, -I,
C₁-C₆ alkyl,
-OH,
-CO-O-R₅₋₂ where R₅₋₂ is C₁-C₄ alkyl or -φ,
-O-R₅₋₂ where R₅₋₂ is as defined above,
-COOH,
-CO-NR₅₋₃R₅₋₄ where R₅₋₃ and R₅₋₄ are the same or different and are -H, C₁-C₃ alkyl, or R₅₋₃ and R₅₋₄ are taken together with the attached nitrogen atom to form a saturated mono-nitrogen C₃-C₆ heterocyclic ring optionally containing -O-,
-C≡N,
-CHOH-CH₃,
-CO-CH₃,
-SH,
-SO-CH₃,
-SO-φ,
-S-CH₃,
-S-φ,
-SO₂-CH₃,
-SO₂-φ,
-SO₃H,
-SO₂-NH₂,
-N₃,
-NO₂,
-NR_{5-5R5-6} where R₅₋₅ and R₅₋₆ are the same or different and are -H and C₁-C₃ alkyl,
-NH-CO-R₅₋₇ where R₅₋₇ is C₁-C₄ alkyl or -φ,
-CO-O-R₅₋₈ where R₅₋₈ is C₁-C₄ alkyl or -φ either optionally substituted with 1 or 2
-F, -Cl, Br, -I,
C₁-C₆ alkyl,
-OH,
-CO-O-R₅₋₂ where R₅₋₂ is as defined above,
-O-R₅₋₂ where R₅₋₂ is as defined above,
-COOH,
-CO-NR₅₋₃R₅₋₄ where R₅₋₃ and R₅₋₄ are as defined above,
-C≡N,
-CHOH-CH₃,
-CO-CH₃,
-SH,
-SO-CH₃,
-SO-φ,
-S-CH₃,
-S-φ,
-SO₂-CH₃,
-SO₂-φ,
-SO₃H,
-SO₂-NH₂,
-N₃,
-NO₂,
-NR_{5-5R5-6} where R₅₋₅ and R₅₋₆ are as defined above,
-NH-CO-R₅₋₇ where R₅₋₇ is as defined above,
-CO-N(R₅₋₉)₂ where R₅₋₉ is -H or R₅₋₈ as defined above and where the R₅₋₉'s can be taken together with the attached nitrogen atom to form a saturated mononitrogen C₃-C₆ heterocyclic ring optionally containing -0- or -NH-,
-CO-CH₂-CN,
-CO-CH₂-OH,
-CO-CH₂-O-φ where φ is optionally substituted with 1-3 -O-CH₃, 1 -NO₂ and 1 -NH₂,
-CO-CH₂-O-R₅₋₁₀ where R₅₋₁₀ is C₁-C₆ alkyl,
-φ optionally substituted with 1-3 -O-CH₃, 1 -NO₂ and -NH₂,
-CO-R₅₋₁₁ where R₅₋₁₁ is C₁-C₆ alkyl, -φ optionally substituted with 1-4 -F, 1-3 -Cl, 1 -OCH₃, -OH, -NH₂, -NO₂, -CO-CH₃, -SO₂-CH₃ and -SO₂-OH,
-CO-CH(NH-CO-R₅₋₁₂)-R₅₋₁₃ where R₅₋₁₃ is -H or -CR₃ and R₅₋₁₂ is C₁-C₆ alkyl, -φ optionally substituted with 1 or 2 -OH, -OCH₃, -NO₂, -NH₂, -Cl, -F, -NH-CH₃ and -N(CH₃)₂,
-CO-CHNH₂-R₅₋₁₄ where R₅₋₁₄ is -CH(CH₃)₂, -CH₂-CH(CH₃)₂, -CHCH₃-CH₂-CH₃, -CH₂-OH, -CH(OH)-CH₃, -CH₂-φ, -CH₂-φ-OH, -CH₂-SH, -CH₂-CH₂-S-CH₃, and -CH₂-COOH,
-SO₂-CH₃,
-SO₂-φ,
-SO₃H, or
and R₆ is -H and C₁-C₃ alkyl; or
(IIIB) where W₁ and W₂ taken together are
-NR₅-N=N- (LIV)
where R₅ is as defined and above;
and pharmaceutically acceptable salts thereof.

2. A compound according to claim 1, where W₁ and W₂ taken together are -NR₅-N=CR₆-.

3. A compound according to claim 1, where W₁ and W₂ taken together are -NR₅-N=N-.

4. A compound according to any preceding claim, where R₂, R₃ and R₄ are all H.

5. A compound according to any preceding claim, where R₁ is H, C₁-C₆ alkyl, C₃-C₆ cycloalkyl, -OCH₃ or -CHCl₂ and R₅ is selected from -CH₃, -CH₂-CH=CH₂, -CH₂-C≡CH, -CHO and -CO-R₅₋₁ where R₅₋₁ is -CH₃, -C₂H₅, -CH(CH₃)₂, -CHCl₂, -CH₂OH, -CH₂-O-CH₃, 2-thiophenyl or cyclopentyl.

6. A compound according to claim 2, which is
3-[5'-(1-acetylindazolyl)]-5β-(acetamidomethyl)oxazolidin-2-one,
3-(5'-indazolyl)-5β-(acetamidomethyl)oxazolidin-2-one,
3-[5'-(1-ethylindazolyl)]-5β-(acetamidomethyl)oxazolidin-2-one, or
3-[5'-(1-n-propylindazolyl)]-5β-(acetamidomethyl)oxazolidin-2-one.

7. Use of a compound according to any preceding claim, for the manufacture of a medicament for use in the treatment of infection caused by gram-positive or anaerobic organisms.

8. A compound according to any of claims 1 to 4, except that R⁵ is H.

## Patentansprüche

1. 3-(stickstoffsubstituiertes)Phenyl-5β-(amidomethyl)oxazolidin-2-on der Formel (LV) 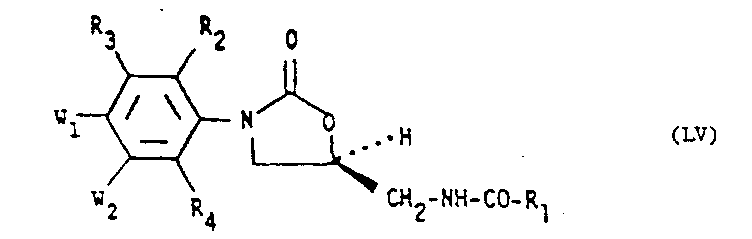 worin bedeuten:
(I) R₁ -H,
C₁-C₁₂-Alkyl, gegebenenfalls substituiert durch 1-3 Cl,
C₃-C₁₂-Cycloalkyl,
C₅-C₁₂-Alkenyl mit einer Doppelbindung,
-φ, gegebenenfalls substituiert durch 1-3 Gruppe(n), ausgewählt aus -OH, -OCH₃, -OC₂H₅, -NO₂, -F, -Cl, -Br, -COOH, -SO₃H und -N(R₁₋₁) (R₁₋₂), worin R₁₋₁ und R₁₋₂ gleich oder verschieden sind und für -H oder C₁-C₂-Alkyl stehen,
Furanyl,
Tetrahydrofuranyl,
2-Thiophen,
Pyrrolidinyl,
Pyridinyl,
-O-R₁₋₃, worin R₁₋₃ für C₁-C₄-Alkyl steht,
-NH₂,
-NHR₁₋₃, worin R₁₋₃ die oben angegebene Bedeutung besitzt,
-NR₁₋₃R₁₋₄, worin R₁₋₄ für C₁-C₃-Alkyl steht und R₁₋₃ die zuvor angegebene Bedeutung besitzt und wobei R₁₋₃ und R₁₋₄ zusammen mit dem daran hängenden Stickstoffatom einen gesättigten, einen Stickstoff enthaltenden C₅-C₇-heterocyclischen Ring einschließlich -O- bilden können,
-CH₂-OH,
-CH₂-OR₁₋₅, worin R₁₋₅ für C₁-C₄-Alkyl oder -CO-R₁₋₆ mit R₁₋₆ gleich C₁-C₄-Alkyl oder -φ steht;
(II) zwei der Reste R₂, R₃ und R₄ -H und der andere der Reste R₂, R₃ und R₄ -H,
-F, -Cl, Br, -I,
C₁-C₆-Alkyl,
-OH,
-CO-O-R₂₋₁, worin R₂₋₁ für C₁-C₄-Alkyl oder -φ steht,
-O-R₂₋₁, worin R₂₋₁ die zuvor angegebene Bedeutung besitzt,
-COOH,
-COO⁻,
-CHO,
-CO-NR₂₋₂R₂₋₃, worin R₂₋₂ und R₂₋₃, die gleich oder verschieden sind, für -H, C₁-C₃-Alkyl stehen oder worin R₂₋₂ und R₂₋₃ zusammen mit dem daran hängenden Stickstoff einen gesättigten, ein Stickstoffatom enthaltenden C₃-C₆-heterocyclischen Ring, gegebenenfalls mit -O- bilden können,
-C≡N,
-C≡CH,
-N≡C,
-CHOH-CH₃,
-CO-CH₃,
-SH,
-SO-CH₃,
-SO-φ,
-S-CH₃,
-S-φ,
-SO₂-CH₃,
-SO₂-φ,
-SO₃H,
-SO₂-NH₂,
-N₃,
-NO₂,
-NR₂₋₄R₂₋₅, worin R₂₋₄ und R₂₋₅, die gleich oder verschieden sein können, für -H und C₁-C₃-Alkyl stehen,
-NH-CO-R₂₋₆, worin R₂₋₆ für C₁-C₄-Alkyl oder -φ steht,
-NH-CO-NH₂,
-CH=CH₂,
-CH₂-CH=CH₂,
-CH=N-OH,
-CH=N-OCH₃, 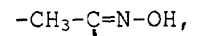 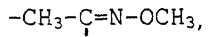 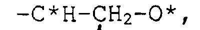 worin die mit dem Sternchen (^{*}) markierten Atome aneinander unter Ringbildung gebunden sind,
(IIIA) W₁ und W₂ zusammengenommen
-NR₅-N=CR₆- (XXXII)
worin bedeuten:
R⁵
C₁-C₁₂-Alkyl,
-CH₂-φ,
-CH₂CH₂-φ,
C₃-C₇-Cycloalkyl,
C₂-C₁₂-Alkenyl mit 1 bis 3 Doppelbindung(en),
C₂-C₁₂-Alkinyl mit einer Dreifachbindung,
-CHO,
-CO-R₅₋₁, worin R₅₋₁ =
(A) C₁-C₆-Alkyl, gegebenenfalls substituiert durch 1 -O-CH₃, -COOH, -NH₂, -SO₃H oder 1-3 -Cl,
(B) C₃-C₇-Cycloalkyl,
(C) 2-Pyridinyl, 2-Thiophen, 2-Thiophenmethyl oder 2-Pyrrol,
(D) -φ, gegebenenfalls substituiert durch 1 bis 3
-F, -Cl, Br, -I,
C₁-C₆-Alkyl,
-OH,
-CO-O-R₅₋₂, worin R₅₋₂ für C₁-C₄-Alkyl oder -φ steht,
-O-R₅₋₂, worin R₅₋₂ die zuvor angegebene Bedeutung besitzt,
-COOH,
-CO-NR₅₋₃R₅₋₄, worin R₅₋₃ und R₅₋₄ gleich oder verschieden sein können und für -H, C₁-C₃-Alkyl stehen oder worin R₅₋₃ und R₅₋₄ zusammen mit dem daran hängenden Stickstoffatom einen gesättigten, ein Stickstoffatom enthaltenden C₃-C₆-heterocyclischen Ring, gegebenenfalls mit -O- bilden können,
-C≡N,
-CHOH-CH₃,
-CO-CH₃,
-SH,
-SO-CH₃,
-SO-φ,
-S-CH₃,
-S-φ,
-SO₂-CH₃,
-SO₂-φ,
-SO₃H,
-SO₂-NH₂,
-N₃,
-NO₂,
-NR₅₋₅R₅₋₆, worin R₅₋₅ und R₅₋₆, die gleich oder verschieden sein können, für -H und C₁-C₃-Alkyl stehen,
-NH-CO-R₅₋₇, worin R₅₋₇ für C₁-C₄-Alkyl oder -φ steht,
-CO-O-R₅₋₈, worin R₅₋₈ für C₁-C₄-Alkyl oder -φ, gegebenenfalls substituiert durch 1 oder 2
-F, -Cl, Br, -I,
C₁-C₆-Alkyl,
-OH,
-CO-O-R₅₋₂, worin R₅₋₂ die zuvor angegebene Bedeutung besitzt,
-O-R₅₋₂, worin R₅₋₂ die zuvor angegebene Bedeutung besitzt,
-COOH,
-CO-NR₅₋₃R₅₋₄, worin R₅₋₃ und R₅₋₄ die zuvor angegebene Bedeutung besitzen,
-C≡N,
-CHOH-CH₃,
-CO-CH₃,
-SH,
-SO-CH₃,
-SO-φ,
-S-CH₃,
-S-φ,
-SO₂-CH₃,
-SO₂-φ,
-SO₃H,
-SO₂NH₂,
-N₃,
-NO₂,
-NR₅₋₅R₅₋₆, worin R₅₋₅ und R₅₋₆ die zuvor angegebene Bedeutung besitzen,
-NH-CO-R₅₋₇, worin R₅₋₇ die zuvor angegebene Bedeutung besitzt, steht,
-CO-N(R₅₋₉)₂, worin R₅₋₉ für -H oder R₅₋₈ in der zuvor angegebenen Bedeutung steht und wo die Reste R₅₋₉ zusammen mit dem daran hängenden Stickstoffatom einen gesättigten, ein Stickstoffatom enthaltenden C₃-C₆-heterocyclischen Ring, gegebenenfalls mit -O- oder -NH- bilden können,
-CO-CH₂-CN,
-CO-CH₂-OH,
-CO-CH₂-O-φ, wobei φ gegebenenfalls durch 1-3 -O-CH₃, 1 -NO₂ und 1 -NH₂ substituiert ist,
-CO-CH₂-O-R₅₋₁₀, worin R₅₋₁₀ für C₁-C₆-Alkyl,
-φ, gegebenenfalls substituiert durch 1-3 -O-CH₃, 1 -NO₂ und -NH₂,
-CO-R₅₋₁₁, mit R₅₋₁₁ gleich C₁-C₆-Alkyl, -φ, gegebenenfalls substituiert durch 1-4 -F, 1-3 -Cl, 1 -OCH₃, -OH, -NH₂, -NO₂, -CO-CH₃, -SO₂-CH₃ und -SO₂-OH, steht,
-CO-CH(NH-CO-R₅₋₁₂)-R₅₋₁₃, worin R₅₋₁₃ für -H oder -CH₃ steht und R₅₋₁₂ C₁-C₆-Alkyl, -φ, gegebenenfalls substituiert mit 1 oder 2 -OH, -OCH₃, -NO₂, -NH₂, -Cl, -F, -NH-CH₃ und -N(CH₃)₂, darstellt,
-CO-CHNH₂-R₅₋₁₄, worin R₅₋₁₄ für -CH(CH₃)₂, -CH₂-CH(CH₃)₂, -CHCH₃-CH₂-CH₃, -CH₂-OH, -CH(OH)-CH₃, -CH₂-φ, -CH₂-φ-OH, -CH₂-SH, -CH₂-CH₂-S-CH₃ und -CH₂-COOH steht,
-SO₂-CH₃,
-SO₂-φ,
-SO₃H, und
R₆ -H und C₁-C₃-Alkyl; oder
(IIIB) W₁ und W₂ zusammengenommen
-NR₅-N=N- (LIV)
worin R₅ die oben angegebene Bedeutung besitzt, und dessen pharmazeutisch akzeptable Salze.

2. Verbindung nach Anspruch 1, wobei W₁ und W₂ zusammen -NR₅-N=CR₆- bedeuten.

3. Verbindung nach Anspruch 1, wobei W₁ und W₂ zusammen -NR₅-N=N- bedeuten.

4. Verbindung nach einem der vorhergehenden Ansprüche, wobei R₂, R₃ und R₄ sämtliche für H stehen.

5. Verbindung nach einem der vorhergehenden Ansprüche, worin R₁ für H, C₁-C₂-Alkyl, C₃-C₆-Cycloalkyl, -OCH₃ oder -CHCl₂ steht und R₅ aus -CH₃, -CH₂-CH=CH₂, -CH₂-C≡CH, -CHO und -CO-R₅₋₁ mit R₅₋₁ gleich -CH₃, -C₂H₅, -CH(CH₃)₂, -CHCl₂, -CH₂OH, -CH₂-O-CH₃, 2-Thiophenyl oder Cyclopentyl ausgewählt ist.

6. Verbindung nach Anspruch 2, nämlich
3-[5'-(1-Acetylindazolyl)]-5β-(acetamidomethyl)-oxazolidin-2-on,
3-(5'-Indazolyl)-5β-(acetamidomethyl)-oxazolidin-2-on,
3-[5'-(1-Ethylindazolyl)]-5β-(acetamidomethyl)-oxazolidin-2-on oder
3-[5'-(1-n-Propylindazolyl)]-5β-(acetamidomethyl)oxazolidin-2-on.

7. Verwendung einer Verbindung nach einem der vorhergehenden Ansprüche zur Herstellung eines Medikaments zur Verwendung bei der Behandlung einer durch grampositive oder anaerobe Organismen hervorgerufenen Infektion.

8. Verbindung nach einem der Ansprüche 1 bis 4, ausgenommen den Fall, dass R⁵ für H steht.

## Revendications

1. 3-(substituant sur l'azote)-phényl-5β-(amidométhyl)oxazolidine-2-one de formule (LV) 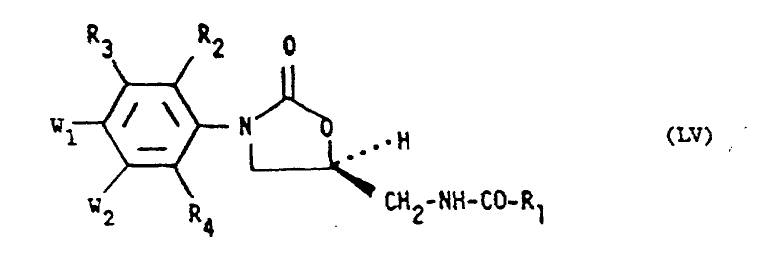 dans laquelle
(I) R₁ représente -H,
un groupe alkyle en C₁ à C₁₂ facultativement substitué avec 1 à 3 substituants Cl,
un groupe cycloalkyle en C₃ à C₁₂,
un groupe alcényle en C₅ à C₁₂ contenant une double liaison,
un groupe -φ facultativement substitué avec 1 à 3 groupes choisis entre des groupes -OH, -OCH₃, -OC₂H₅, -NO₂, -F, -Cl, -Br, -COOH, -SO₃H et -N(R₁₋₁)(R₁₋₂) dans lesquels R₁₋₁ et R₁₋₂ sont identiques ou différents et représentent -H ou un groupe alkyle en C₁ ou C₂,
un groupe furannyle,
un groupe tétrahydrofurannyle,
un groupe 2-thiophène,
un groupe pyrrolidinyle,
un groupe pyridinyle,
un groupe -O-R₁₋₃ dans lequel R₁₋₃ représente un groupe alkyle en C₁ à C₄,
un groupe -NH₂,
un groupe -NHR₁₋₃ dans lequel R₁₋₃ répond à la définition précitée,
un groupe -NR₁₋₃R₁₋₄ dans lequel R₁₋₄ représente un groupe alkyle en C₁ à C₃ et R₁₋₃ répond à la définition précitée, et dans lequel R₁₋₃ et R₁₋₄ peuvent être pris conjointement avec l'atome d'azote auquel ils sont fixés pour former un noyau hétérocyclique monoazoté saturé en C₅ à C₇ comprenant -O-,
un groupe -CH₂-OH,
un groupe -CH₂OR₁₋₅ dans lequel R₁₋₅ représente un groupe alkyle en C₁ à C₄ ou -CO-R₁₋₆ dans lequel R₁₋₆ représente un groupe alkyle en C₁ à C₄ ou -φ ;
(II) deux des groupes R₂, R₃ et R₄ représentent -H et l'autre des groupes R₂, R₃ et R₄ représente -H,
un groupe -F, -Cl, Br ou -I,
un groupe alkyle en C₁ à C₆,
un groupe -OH,
un groupe CO-O-R₂₋₁ dans lequel R₂₋₁ représente un groupe alkyle en C₁ à C₄ ou -φ,
un groupe -O-R₂₋₁ dans lequel R₂₋₁ répond à la définition précitée,
un groupe -COOH,
un groupe -COO⁻,
un groupe -CHO,
un groupe -CO-NR₂₋₂-R₂₋₃ dans lequel R₂₋₂ et R₂₋₃ sont identiques ou différents et représentent -H ou un groupe alkyle en C₁ à C₃, ou bien R₂₋₂ et R₂₋₃ sont pris conjointement avec l'atome d'azote auquel ils sont fixés pour former un noyau hétérocyclique monoazoté saturé en C₃ à C₆ contenant facultativement -O-,
un groupe -C=N,
un groupe -C=CH,
un groupe -N=C
un groupe -CHOH-CH₃,
un groupe -CO-CH₃,
un groupe -SH,
un groupe -SO-CH₃,
un groupe -SO-φ,
un groupe -S-CH₃,
un groupe -S-φ,
Un groupe -SO₂-CH₃,
un groupe -SO₂-φ,
un groupe -SO₃H,
un groupe -SO₂-NH₂,
un groupe -N₃,
un groupe -NO₂,
un groupe -NR₂₋₄R₂₋₅ dans lequel R₂₋₄ et R₂₋₅ sont identiques ou différents et représentent -H ou un groupe alkyle en C₁ à C₃,
un groupe -NH-CO-R₂₋₆ dans lequel R₂₋₆ représente un groupe alkyle en C₁ à C₄ ou -φ
un groupe -NH-CO-NH₂,
un groupe -CH=CH₂,
un groupe -CH₂-CH=CH₂,
un groupe -CH=N-OH,
un groupe -CH=N-OCH₃, 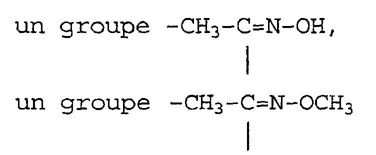
un groupe -C*H-CH₂-O* dans lequel les atomes marqués d'un astérisque (*) sont liés l'un à l'autre, avec pour résultat la formation d'un noyau,
(IIIA) W₁ et W₂, pris conjointement, représentent un groupe
-NR₅-N=CR₆- (XXXII)
dans lequel
R₅ représente
un groupe alkyle en C₁ à C₁₂,
un groupe -CH₂-φ,
un groupe -CH₂CH₂-φ,
un groupe cycloalkyle en C₃ à C₇,
un groupe alcényle en C₂ à C₁₂ contenant 1 à 3 doubles liaisons,
un groupe alcynyle en C₂ à C₁₂ contenant une triple liaison,
un groupe -CHO,
un groupe -CO-R₅₋₁, dans lequel R₅₋₁ représente
(A) un groupe alkyle en C₁ à C₆ facultativement substitué avec un groupe -O-CH₃, -COOH, -NH₂, -SO₃H ou avec 1 à 3 groupes -Cl,
(B) un groupe cycloalkyle en C₃ à C₇,
(C) un groupe 2-pyridinyle, 2-thiophène, 2-thiophèneméthyle ou 2-pyrrole,
(D) un groupe -φ facultativement substitué avec 1 à 3 groupes
-F, -Cl, Br, -I,
alkyle en C₁ à C₆,
-OH,
-CO-O-R₅₋₂ dans lequel R₅₋₂ représente un groupe alkyle en C₁ à C₄ ou -φ,
-O-R₅₋₂ dans lequel R₅₋₂ répond à la définition précitée,
-COOH,
-CO-NR₅₋₃R₅₋₄ dans lequel R₅₋₃ et R₅₋₄ sont identiques ou différents et représentent -H ou un groupe alkyle en C₁ à C₄, ou bien R₅₋₃ et R₅₋₄ sont pris conjointement avec l'atome d'azote auquel ils sont fixés pour former un noyau hétérocyclique monoazoté saturé en C₃ à C₆ contenant facultativement -O-,
-C=N,
-CHOH-CH₃,
-CO-CH₃,
-SH,
-SO-CH₃,
-SO-φ,
-S-CH₃,
-S,φ,
-SO₂-CH₃
-SO₂-φ,
-SO₃H,
-SO₂-NH₂,
-N₃,
-NO₂,
-NR₅₋₅R₅₋₆ dans lequel R₅₋₅ et R₅₋₆ sont identiques ou différents et représentent -H ou un groupe alkyle en C₁ à C₃,
-NH-CO-R₅₋₇ dans lequel R₅₋₇ représente un groupe alkyle en C₁ à C₄ ou -φ,
un groupe -CO-O-R₅₋₈ dans lequel R₅₋₈ représente un groupe alkyle en C₁ à C₄ ou -φ facultativement substitué avec un ou deux groupes
-F, -Cl, Br, -I,
alkyle en C₁ à C₆,
-OH,
-CO-O-R₅₋₂ dans lequel R₅₋₂ répond à la définition précitée,
-O-R₅₋₂ dans lequel R₅₋₂ répond à la définition précitée,
-COOH,
-CO-NR₅₋₃R₅₋₄ dans lequel R₅₋₃ et R₅₋₄ répondent aux définitions précitées,
-C=N,
-CHOH-CH₃,
-CO-CH₃,
-SH,
-SO-CH₃,
-SO-φ,
-S-CH₃,
-S-φ,
-SO₂-CH₃,
-SO₂-φ,
-SO₃H,
-SO₂-NH₂,
-N₃,
-NO₂,
-NR₅₋₅R₅₋₆ dans lequel R₅₋₅ et R₅₋₆ répondent aux définitions précitées,
-NH-CO-R₅₋₇ dans lequel R₅₋₇ répond à la définition précitée,
un groupe -CO-N(R₅₋₉)₂ dans lequel R₅₋₉ représente -H ou un groupe R₅₋₈ répondant à la définition précitée et dans lequel les groupes R₅₋₉ peuvent être pris conjointement avec l'atome d'azote auquel ils sont fixés pour former un noyau hétérocyclique monoazoté saturé en C₃ à C₆ contenant facultativement -O- ou -NH-,
un groupe -CO-CH₂-CN,
un groupe -CO-CH₂-OH,
un groupe -CO-CH₂-O-φ dans lequel φ est facultativement substitué avec 1 à 3 groupes -O-CH₃, un groupe -NO₂ et un groupe -NH₂,
un groupe -CO-CH₂-O-R₅₋₁₀ dans lequel R₅₋₁₀ représente un groupe alkyle en C₁ à C₆,
un groupe -φ facultativement substitué avec 1 à 3 groupes -O-CH₃, un groupe -NO₂ et un groupe -NH₂,
un groupe -CO-R₅₋₁₁ dans lequel R₅₋₁₁ représente un groupe alkyle en C₁ à C₆, -φ facultativement substitué avec 1 à 4 groupes -F, 1 à 3 groupes -Cl, un groupe -OCH₃, -OH, -NH₂, -NO₂, -CO-CH₃, -SO₂-CH₃ ou -SO₂-OH,
un groupe -CO-CH(NH-CO-R₅₋₁₂) -R₅₋₁₃ dans lequel R₅₋₁₃ représente -H ou un groupe -CH₃ et R₅₋₁₂ représente un groupe alkyle en C₁ à C₆, -φ facultativement substitué avec 1 ou 2 groupes -OH, -OCH₃, -NO₂, -NH₂, -Cl, -F, -NH-CH₃ et -N(CH₃)₂,
un groupe -CO-CHNH₂-R₅₋₁₄ dans lequel R₅₋₁₄ représente un groupe -CH(CH₃)₂, -CH₂-CH(CH₃)₂, -CHCH₃-CH₂-CH₃, -CH₂-OH, -CH(OH)-CH₃, -CH₂-φ, -CH₂-φ-OH, -CH₂-SH, -CH₂-CH₂-S-CH₃ et -CH₂-COOH,
un groupe -SO₂-CH₃,
un groupe -SO₂-φ,
un groupe -SO₃H,
et R₆ représente -H ou un groupe alkyle en C₁ à C₃ ; ou
(IIIB) W₁ et W₂, pris conjointement, représentent un groupe
-NR₅-N=N (LIV)
dans lequel R₅ répond à la définition précitée ;
et ses sels pharmaceutiquement acceptables.

2. Composé suivant la revendication 1, dans lequel W₁ et W₂, pris conjointement, représentent un groupe -NR₅-N=CR₆-.

3. Composé suivant la revendication 1, dans lequel W₁ et W₂, pris conjointement, représentent un groupe -NR₅-N=N-.

4. Composé suivant l'une quelconque des revendications précédentes, dans lequel R₂, R₃ et R₄ représentent tous H.

5. Composé suivant l'une quelconque des revendications précédentes, dans lequel R₁ représente H, un groupe alkyle en C₁ à C₆, cycloalkyle en C₃ à C₆, -OCH₃ ou -CHCl₂ et R₅ est choisi entre des groupes -CH₃, -CH₂-CH=CH₂, -CH₂-C≡CH, -CHO et -CO-R₅₋₁ dans lequel R₅₋₁ représente un groupe -CH₃, -C₂H₅, -CH(CH₃)₂, -CHCl₂, -CH₂OH, -CH₂-O-CH₃, 2-thiophényle ou cyclopentyle.

6. Composé suivant la revendication 2, qui consiste en
3- [5'-(1-acétylindazolyl)]-5β-(acétamidométhyl)oxazolidine-2-one,
3-(5'-indazolyl)-5β-(acétamidométhyl)oxazolidine-2-one,
3-[5'-(1-éthylindazolyl)]-5β-(acétamidométhyl)oxazolidine-2-one, ou
3-[5'-(1-n-propylindazolyl)]-5β-(acétamidométhyl)oxazolidine-2-one.

7. Utilisation d'un composé suivant l'une quelconque des revendications précédentes, pour la production d'un médicament destiné à être utilisé dans le traitement d'une infection provoquée par des organismes Gram-positifs ou anaérobies.

8. Composé suivant l'une quelconque des revendications 1 à 4, sauf que R₅ représente H.
